# FASCICULE DE BREVET EUROPEEN

(11) **EP 1 778 797 B1**
(45) Date de publication et mention de la délivrance du brevet: **16.02.2011**
(21) Numéro de dépôt: 05786142.9
(22) Date de dépôt: 05.07.2005
(51) Int. Cl.: C09D 7/00, C09D 17/00, D06P 1/52

(54) **UTILISATION DE COPOLYMERES ACRYLIQUES HYDROSOLUBLES DANS DES FORMULATIONS AQUEUSES EVENTUELLEMENT PIGMENTEES ET FORMULATIONS OBTENUES**
VERWENDUNG VON WASSERLÖSLICHEN ACRYLCOPOLYMEREN IN OPTISCH PIGMENTIERTEN WÄSSRIGEN FORMULIERUNGEN UND DARAUS ERHALTENE FORMULIERUNGEN
USE OF WATER-SOLUBLE ACRYLIC COPOLYMERS IN OPTIONALLY PIGMENTED AQUEOUS FORMULATIONS AND RESULTING FORMULATIONS

(30) Priorité: 08.07.2004 FR 0407570
(43) Date de publication de la demande: 02.05.2007
(73) Titulaire: COATEX S.A.S., 69730 Genay (FR)
(72) Inventeur: SUAU, Jean-Marc, F-69480 Lucenay (FR); KENSICHER, Yves, F-69620 Theize (FR); RUHLMANN, Denis, F-69730 Genay (FR)
(74) Mandataire: Richebourg, Michel François
(86) Numéro de dépôt international: PCT/FR2005/001714
(87) Numéro de publication internationale: WO 2006/016035

(56) Documents cités:
- EP-A- 1 403 347
- FR-A- 2 693 203
- US-A1- 2003 202 953

## Description

La présente invention concerne le secteur des formulations aqueuses éventuellement pigmentées.

L'invention concerne tout d'abord l'utilisation comme épaississant, dans des formulations aqueuses éventuellement pigmentées, telles que les formulations de revêtement comme les peintures en phase aqueuse et notamment les peintures dispersions, les vernis, les sauces de couchage papetières, la cosmétique, la détergence, les formulations textiles et les boues de forage, d'un copolymère acrylique hydrosoluble constitué d'au moins un monomère à insaturation éthylénique et à fonction carboxylique, d'au moins un monomère non ionique à insaturation éthylénique, d'au moins un monomère oxyalkylé à insaturation éthylénique terminé par une chaîne ramifiée hydrophobe et non aromatique comportant de 10 à 24 atomes de carbone, et éventuellement d'au moins un monomère appelé monomère réticulant, comportant au moins deux insaturations éthyléniques.

L'invention concerne également l'épaississant permettant d'obtenir un développement et/ou un maintien optimaux de la viscosité des formulations aqueuses en présence de composés hydrosolubles ou non ou lors de l'addition des dits composés dans les dites formulations aqueuses.

L'invention concerne aussi le procédé de fabrication des dites formulations aqueuses éventuellement pigmentées.

L'invention concerne enfin les formulations aqueuses éventuellement pigmentées obtenues selon l'invention.

Dans le domaine des formulations aqueuses contenant éventuellement une ou plusieurs charges minérales, plus particulièrement dans le secteur des peintures en phase aqueuse et notamment les peintures dispersions, maîtriser la rhéologie desdites formulations, tant au stade de la fabrication, que pendant leur transport, leur stockage ou lors de leur mise en oeuvre est un des objectifs fondamentaux pour l'homme du métier. La diversité des contraintes pratiques au niveau de chacune de ces étapes renvoie à une multiplicité de comportements rhéologiques différents. On peut néanmoins résumer le besoin de l'homme du métier à l'obtention d'un effet d'épaississement de la formulation aqueuse, tant pour des raisons de stabilité au cours du temps, que pour une possible application de la peinture sur une surface verticale, l'absence d'éclaboussures au moment de la mise en oeuvre, ou de coulure après la mise en oeuvre, etc... De fait, on a désigné les produits qui contribuent à cette régulation du comportement rhéologique sous le terme d'épaississants.

Historiquement, on a utilisé dès les années 1950 des gommes et des épaississants à base cellulosique, dont une des caractéristiques essentielles est leur poids moléculaire élevé. Ces composés présentent cependant un certain nombre d'inconvénients tels que leur instabilité au cours du temps comme le rappelle le document US 4 673 518, la nécessité d'en utiliser une quantité importante mise en avant dans le document EP 0 250 943, et les coûts de production qu'ils engendrent (notamment au niveau du traitement des déchets) tel qu'indiqué dans le document US 4 384 096.

L'homme du métier s'est ensuite tourné vers une nouvelle catégorie d'épaississants dits associatifs, dont le mécanisme de fonctionnement et les caractéristiques sont aujourd'hui bien connus et rappelés dans le document « Rheology modifiers for water-borne paints » (Surface Coatings Australia, Octobre 1985, pp 6). Il s'agit de polymères hydrosolubles disposant de groupements hydrophobes insolubles et créant l'effet associatif par interaction de ces groupements hydrophobes avec la surface des particules de liant en dispersion menant ainsi à un réseau tridimensionnel qui provoque l'augmentation de la viscosité du milieu.

On peut distinguer de manière très schématique parmi les polymères synthétiques les épaississants associatifs polyuréthannes, et les épaississants associatifs acryliques.

De tels épaississants sont aujourd'hui couramment utilisés entre autre dans le secteur des peintures en phase aqueuse et notamment les peintures dispersions. Toutefois, leur utilisation n'est pas dénuée d'inconvénients dont deux, majeurs, que la Demanderesse tient ici à développer. Le premier concerne la « sensibilité aux composés hydrosolubles ou non » (et notamment les sels et les tensio-actifs), et le second la « compatibilité pigmentaire » ou « acceptance de couleur ». On rencontre ces problèmes dans un grand nombre de formulations aqueuses, telles que les peintures en phase aqueuse et notamment les peintures dispersions, mais aussi telles que les formulations cosmétiques ou détergentes. Chacun de ces problèmes va par la suite être envisagé sous l'angle des solutions proposées par l'état de la technique, tant du point de vue des épaississants acryliques associatifs que des épaississants polyuréthannes associatifs.

On désigne par « sensibilité aux composés hydrosolubles ou non» le comportement d'un épaississant qui, lorsqu'il est mis en présence de tels composés, et notamment de sels et de tensio-actifs, tend à ne plus maintenir la viscosité à un degré satisfaisant pour l'homme du métier ou l'utilisateur de ces formulations aqueuses. La perte de viscosité résultante est préjudiciable aux qualités de stockage et d'application desdites formulations. Ce type de phénomène est largement reporté dans la littérature, tout particulièrement pour les épaississants acryliques associatifs, comme le souligne le document EP 0 013 836, qui relate la sensibilité aux sels et notamment au chlorure de sodium de tels additifs. Ce document enseigne à l'homme du métier la fabrication d'épaississants acryliques associatifs à partir d'un monomère choisi parmi l'acide acrylique et l'acide méthacrylique, d'un monomère qui est un ester d'alcoyle, et d'un monomère hydrophobe comportant un radical ayant de 8 à 30 atomes de carbone. Sa principale fonction est d'améliorer le profil rhéologique des peintures aqueuses dans lesquelles il est utilisé. De même, le document EP 0011 806 enseigne la fabrication d'autres épaississants acryliques associatifs, à partir d'un monomère carboxylique, d'acrylate d'éthyle, et d'un monomère hydrophobe comportant un radical alkyle ou alkyle phényle ayant de 8 à 20 atomes de carbone. Enfin, le document EP 0 577 526 décrit des épaississants acryliques associatifs à base d'un monomère carboxylique, d'un monomère à insaturation éthylénique mais non carboxylé, et d'un monomère oxyalkylé terminé par une chaîne grasse hydrophobe. Néanmoins, en terme d'insensibilité aux sels, ces copolymères ne donnent pas satisfaction à l'homme du métier.

Un autre problème auquel l'homme du métier doit faire face est celui de la compatibilité pigmentaire. En matière de formulation de peintures, on désigne sous le terme « base » ou « base blanche », la composition aqueuse pigmentée et chargée de couleur blanche, qui sert de base à la composition pigmentaire colorée ou pigmentée finale.

Cette composition aqueuse chargée et/ou pigmentée de couleur blanche est formée d'une phase liquide qui peut être de l'eau ou un solvant organique miscible à l'eau ou encore un mélange des deux, d'un ou plusieurs polymères en solution ou en suspension dans la phase liquide dénommés « liants », des charges et/ou pigments, d'au moins un agent dispersant des charges et/ou pigments qui peut être un polymère ou un copolymère hydrosoluble, des adjuvants aussi divers que des agents de coalescence, des biocides, des anti-mousses ou autres, et enfin d'au moins un agent épaississant qui est un polymère ou un copolymère naturel ou de synthèse. Lorsqu'on ajoute auxdites « bases » de couleur blanche les pigments colorés, sous forme de concentrés pigmentaires ou pâtes pigmentaires, on obtient la formulation pigmentaire résultante.

La composition des concentrés de pigments colorés ou concentrés pigmentaires ou pâtes pigmentaires, comprend en général également des additifs ou composés du type tensio-actif ou sel électrolyte ou co-solvant miscible à l'eau en quantité importante.

Les concentrés pigmentaires les plus employés sont dits universels car ils peuvent être ajoutés non seulement à des peintures en phase aqueuse mais aussi à des peintures en phase solvant.

En pratique, si la formulation finale présente une compatibilité pigmentaire insuffisante, d'une part il peut y avoir une incidence sur la rhéologie de la formulation pigmentaire résultante et d'autre part il peut y avoir une incidence sur la force colorante développée par le ou les pigments du ou des concentrés pigmentaires dans la formulation pigmentaire résultante.

Cette perte de force colorante peut conduire à un film de peinture dont la teinte est uniformément plus « délavée », salie ou dégradée au blanc par rapport à la référence. Evidemment, cette perte de force colorante est très préjudiciable à la mise à la teinte d'une peinture par un automate d'après la mesure d'un spectrophotocolorimètre, telle qu'elle se pratique dans les grandes surfaces de bricolage ou chez les détaillants de peinture.

En effet, l'ordinateur calculera qu'une certaine quantité de concentrés pigmentaires universels devrait permettre d'approcher la teinte désirée mais la perte de force colorante liée à cette mauvaise compatibilité faussera le résultat.

Ce phénomène peut être mesuré par l'utilisation d'un spectrophotocolorimètre permettant de mesurer les coordonnées tri-chromatiques (Huntsmann : L*,a*,b*) donc la couleur d'un film de peinture sec.

L* donnera une valeur dans l'échelle des gris (plus L* est grand et positif , plus le film est clair; plus L* est petit et négatif, plus le film est foncé). La deuxième coordonnée, a*, caractérise la couleur dans une échelle qui va du vert au rouge (plus a* est grand et positif , plus le film est rouge ; plus a* est petit et négatif, plus le film est vert). La troisième coordonnée, b*, caractérise la couleur dans une échelle qui va du bleu au jaune (plus b* est grand et positif , plus le film est jaune ; plus b* est petit et négatif, plus le film est bleu).

Dans certains cas, plus rares, on observe même des variations de teintes par zones dans un même film de peinture. Ces variations sont liées aux différences de cisaillement subies par ces différentes zones lors de l'application du film de peinture avec un outil tel que par exemple un pinceau. Les pigments du colorant universel qui sont dans un état de mauvaise dispersion dans la peinture, vont exprimer une force colorante qui dépendra de la force du cisaillement subie par la peinture qui les contient. Dans le cas de l'utilisation d'un pinceau, ce phénomène soulignera les marques du pinceau (là où les poils du pinceau appuient le plus fort) en leur donnant une couleur différente du reste du film de peinture. Pour visualiser ce phénomène, l'homme de l'art peut appliquer au pinceau ou utiliser un test dit test du rub-out consistant à appliquer un film de peinture uniformément, sans différence de cisaillement, à l'aide d'une cale puis à cisailler une partie du film de peinture encore frais, non sec, avec un doigt. La zone cisaillée peut devenir plus claire ou plus foncée que la zone non cisaillée.

Dans cette lignée, l'homme du métier connaît le document US 5 973 063 qui décrit un épaississant associatif polyuréthanne ainsi que le document FR 2 826 014 qui enseigne qu'il est possible, en sélectionnant un composé à base de polyisocyanate ayant en bout de chaîne des groupements hydrocarbonés comportant au moins trois cycles aromatique, de fabriquer un épaississant polyuréthanne présentant une bonne compatibilité pigmentaire, et développant une viscosité élevée sous bas gradient de cisaillement.

Par conséquent, l'homme du métier, qui recherche à mettre à disposition de l'utilisateur final une composition aqueuse dont le profil rhéologique reste acceptable en présence de composés hydrosolubles ou non hydrosolubles ou lors de l'addition des dits composés comme les sels ou les tensio-actifs dans le domaine de la détergence ou de la cosmétique, ou encore comme les concentrés pigmentaires universels dans le domaine de la peinture, notamment sous bas gradient de cisaillement pour obtenir un bon étalement de la peinture, se trouve confronté aux problèmes de la forte baisse de la viscosité de la formulation en présence des dits composés, et s'oriente de manière naturelle vers les épaississants associatifs de type polyuréthanne. Ceux-ci, comme on vient de le voir, sont bien connus pour offrir une bonne résistance aux sels et développer une viscosité appropriée sous faible cisaillement.

Le dernier document FR 2826014 a finalement démontré que la «voie polyuréthanne » autorise également la fabrication de tels épaississants présentant une compatibilité pigmentaire satisfaisante.

Or, poursuivant ses recherches en vue d'améliorer la stabilité de la viscosité des formulations aqueuses en présence de composés hydrosolubles ou non hydrosolubles ou lors de l'addition des dits composés dans les dites formulations aqueuses, ainsi que d'améliorer la compatibilité pigmentaire, tout en maintenant une viscosité de la formulation acceptable pour l'utilisateur final, la Demanderesse a trouvé de manière tout à fait surprenante que l'ensemble de ces problèmes peut être résolu par la sélection du monomère oxyalkylé à insaturation éthylénique du copolymère qui est un épaississant acrylique associatif et par sa mise en oeuvre comme additif dans la formulation.

Ce copolymère acrylique hydrosoluble est constitué d'au moins un monomère à insaturation éthylénique et à fonction carboxylique, d'au moins un monomère non ionique à insaturation éthylénique, d'au moins un monomère oxyalkylé à insaturation éthylénique terminé par une chaîne ramifiée hydrophobe et non aromatique comportant de 10 à 24 atomes de carbone et éventuellement d'au moins un monomère appelé monomère réticulant, comportant au moins deux insaturations éthyléniques.

L'objet de l'invention est donc l'utilisation dans des formulations aqueuses éventuellement pigmentées, telles que les formulations de revêtement comme les peintures en phase aqueuse et notamment les peintures dispersions, les vernis, les sauces de couchage papetières, la cosmétique, la détergence, les formulations textiles et les boues de forage, de ce copolymère en tant qu'épaississant permettant d'obtenir un développement et/ou un maintien de la viscosité des formulations aqueuses en présence de composés hydrosolubles ou non ou lors de l'addition des dits composés dans les dites formulations aqueuses.

Un autre objet de l'invention est l'épaississant constitué de ce copolymère acrylique hydrosoluble constitué d'au moins un monomère à insaturation éthylénique et à fonction carboxylique, d'au moins un monomère non ionique à insaturation éthylénique, d'au moins un monomère oxyalkylé à insaturation éthylénique terminé par une chaîne ramifiée hydrophobe et non aromatique comportant de 10 à 24 atomes de carbone et éventuellement d'au moins un monomère appelé monomère réticulant, comportant au moins deux insaturations éthyléniques.

Un autre objet de l'invention est le procédé de fabrication des formulations aqueuses éventuellement pigmentées.

Un dernier objet de l'invention réside dans les formulations aqueuses éventuellement pigmentées obtenues selon l'invention.

Ces formulations aqueuses éventuellement pigmentées contenant l'épaississant selon l'invention sont les formulations aqueuses de revêtement choisies parmi les peintures dispersions, les vernis, les sauces de couchage, les formulations cosmétiques, les formulations détergentes, les formulations (de préparation, d'impression ou de teinture) textiles et les boues de forage.

Ces formulations sont également des formulations pour plaques de plâtre telles que les formulations de pâte à joint pour plaques de plâtre, les formulations pour céramique, les formulations pour cuir, les formulations de plâtre ou encore les formulations pour liants hydrauliques telles que les formulations de mortier.

Selon l'invention, les copolymères ajoutés dans les formulations aqueuses comme épaississants permettent de conférer aux formulations aqueuses éventuellement pigmentées un comportement rhéologique satisfaisant en vue de leur application, et notamment une augmentation de leur viscosité sous moyen gradient de cisaillement.

La Demanderesse indique qu'on entend dans la présente demande sous l'expression « moyen gradient de cisaillement » un domaine de gradient de cisaillement encore appelé gradient de vitesse compris entre 20 s⁻¹ et 1000 s⁻¹.

De plus, ces copolymères développent une excellente tolérance vis-à-vis des sels et des tensio-actifs que peuvent contenir ces formulations. Enfin, de tels copolymères confèrent aux formulations aqueuses dans lesquelles ils sont utilisés une très bonne compatibilité pigmentaire.

Ces buts sont atteints grâce à l'utilisation, comme épaississant permettant d'obtenir un développement et/ou un maintien de la viscosité des formulations aqueuses en présence de composés hydrosolubles ou non ou lors de l'addition des dits composés dans les dites formulations aqueuses, de copolymères acryliques hydrosolubles caractérisés en ce que ledit copolymère est constitué :
a) d'au moins un monomère à insaturation éthylénique et à fonction carboxylique,
b) d'au moins un monomère non ionique à insaturation éthylénique,
c) d'au moins un monomère oxyalkylé à insaturation éthylénique terminé par une chaîne ramifiée hydrophobe et non aromatique comportant de 10 à 24 atomes de carbone,
d) et éventuellement d'au moins un monomère appelé monomère réticulant, comportant au moins deux insaturations éthyléniques.

Ce copolymère épaississant est également caractérisé en ce que :
a) le ou les monomères à insaturation éthylénique et à fonction carboxylique sont choisis parmi les monomères à insaturation éthylénique et à fonction monocarboxylique tels que l'acide acrylique ou méthacrylique, ou encore les hémiesters de diacides tels que les monoesters en C₁ à C₄ des acides maléique ou itaconique, ou leurs mélanges, ou choisi parmi les monomères à insaturation éthylénique et fonction dicarboxylique tels que l'acide crotonique, isocrotonique, cinnamique, itaconique, maléique, ou encore les anhydrides d'acides carboxyliques, tels que l'anhydride maléique,
b) le ou les monomères non ioniques à insaturation éthylénique sont choisis parmi les esters, les amides ou les nitriles des acides acrylique et méthacrylique, tels que les acrylates ou méthacrylates de méthyle, éthyle, butyle, 2-éthyle-hexyle, ou parmi l'acrylonitrile, l'acétate de vinyle, le styrène, le méthylstyrène, le diisobutylène, la vinylpyrrolidone, la vinylcaprolactame,
c) le ou les monomères oxyalkylé à insaturation éthylénique et terminés par une chaîne ramifiée hydrophobe et non aromatique comportant de 10 à 24 atomes de carbone, possèdent la formule suivante : 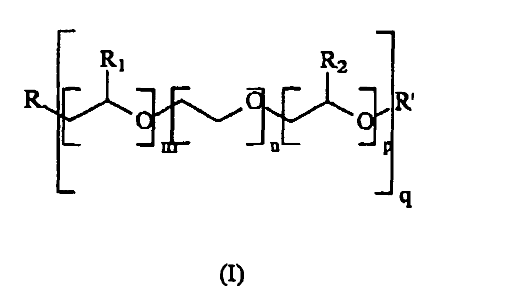 dans laquelle :
   - m et p représentent un nombre de motifs d'oxyde d'alkylène allant de 0 à 150,
   - n représente un nombre de motifs d'oxyde d'éthylène allant de 5 à 150,
   - q représente un nombre entier au moins égal à 1 et tel que 5 ≤ (m+n3-p)q ≤ 150, et préférentiellement tel que 15≤ (m+n+p)q ≤ 120,
   - R₁ représente le radical méthyle ou éthyle,
   - R₂ représente le radical méthyle ou éthyle,
   - R représente un radical contenant une fonction insaturée polymérisable, appartenant au groupe des esters acrylique, méthacrylique, maléique, itaconique, ou crotonique,
   - R' représente une chaîne ramifiée hydrophobe et non aromatique comportant de 10 à 24 atomes de carbone, et possède 2 ramifications comportant au moins 6 atomes de carbone.
d) le ou les monomères éventuels, appelés monomères réticulants, comportant au moins deux insaturations éthyléniques sont notamment choisis dans le groupe constitué par le diacrylate d'éthylène glycol, le diméthacrylate d'éthylène glycol, le triméthylolpropanetriacrylate, le triméthylolpropanetriméthacrylate, l'acrylate d'allyle, le méthylène-bis-acrylamide, le méthylène-bis-méthacrylamide, le tétrallyloxyéthane, les triallylcyanurates, les éthers allyliques obtenus à partir de polyols choisis parmi le pentaérythritol, le sorbitol, ou encore le sucrose.

De manière plus préférentielle, ledit copolymère est caractérisé en ce que la chaîne ramifiée hydrophobe et non aromatique comporte de 16 à 20 atomes de carbone, et possède 2 ramifications comportant au moins 6 atomes de carbone.

De manière encore plus préférentielle, la chaîne ramifiée hydrophobe non aromatique est choisie parmi le 2-hexyl 1-decanyle et le 2-octyl 1-dodecanyle.

Ce copolymère utilisé comme épaississant selon l'invention est également caractérisé en ce qu'il contient, exprimé en poids des monomères :
a) de 2 à 95 %, et préférentiellement de 5 à 90 % d'au moins un monomère à insaturation éthylénique et à fonction carboxylique choisis parmi les monomères à insaturation éthylénique et à fonction monocarboxylique tels que l'acide acrylique ou méthacrylique, ou encore les hémiesters de diacides tels que les monoesters en C₁ à C₄ des acides maléique ou itaconique, ou leurs mélanges, ou choisi parmi les monomères à insaturation éthylénique et fonction dicarboxylique tels que l'acide crotonique, isocrotonique, cinnamique, itaconique, maléique, ou encore les anhydrides d'acides carboxyliques, tels que l'anhydride maléique,
b) de 2 à 95 %, et préférentiellement de 5 à 90 % d'au moins un monomère à insaturation éthylénique et sans fonction carboxylique choisis parmi les esters, les amides ou les nitriles des acides acrylique et méthacrylique, tels que les acrylates ou méthacrylates de méthyle, éthyle, butyle, 2-éthyle-hexyle, ou par l'acrylonitrile, l'acétate de vinyle, le styrène, le méthylstyrène, le diisobutylène, la vinylpyrrolidone, la vinylcaprolactame,
c) de 2 à 25 % et préférentiellement de 5 à 20 % d'au moins un monomère à insaturation éthylénique et terminé par une chaîne ramifiée hydrophobe et non aromatique comportant de 10 à 24 atomes de carbone et possède 2 ramifications comportant au moins 6 atomes de carbone, possédant la formule suivante : 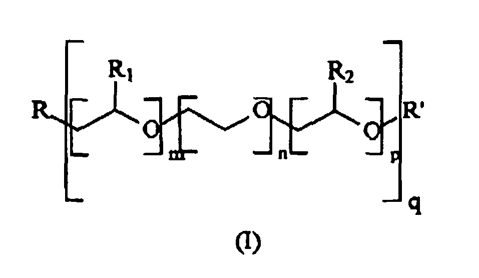 dans laquelle :
   m et p représentent un nombre de motifs d'oxyde d'alkylène allant de 0 à 150,
      - n représente un nombre de motifs d'oxyde d'éthylène allant de 5 à 150,
      - q représente un nombre entier au moins égal à 1 et tel que 5 ≤ (m+n+p)q ≤ 150, et préférentiellement tel que 15 ≤ (m+n+p)q ≤ 120,
      - R₁ représente le radical méthyle ou éthyle,
      - R₂ représente le radical méthyle ou éthyle,
      - R représente un radical contenant une fonction insaturée polymérisable, appartenant au groupe des esters acrylique, méthacrylique, maléique, itaconique, ou crotonique,
      - R' représente une chaîne ramifiée hydrophobe et non aromatique comportant de 10 à 24 atomes de carbone et possède 2 ramifications comportant au moins 6 atomes de carbone.
d) de 0 % à 3 %, d'au moins un monomère comportant au moins deux insaturations éthyléniques choisis dans le groupe constitué par le diacrylate d'éthylène glycol, le diméthacrylate d'éthylène glycol, le triméthylolpropanetriacrylate, le triméthylolpropanetriméthacrylate, l'acrylate d'allyle, le méthylène-bis-acrylamide, le méthylène-bis-méthacrylamide, le tétrallyloxyéthane, les triallylcyanurates, les éthers allyliques obtenus à partir de polyols choisis parmi le pentaérythritol, le sorbitol, ou encore le sucrose,
   le total en poids des monomères a), b), c) et d) étant égal à 100 %.
De manière préférentielle, ce copolymère utilisé selon l'invention comme épaississant est caractérisé en ce qu'il contient, exprimé en poids, de 2 à 25 % et préférentiellement de 5 à 20 % d'au moins un monomère oxyalkylé à insaturation éthylénique et terminé par une chaîne ramifiée hydrophobe et non aromatique comportant de 12 à 24 atomes de carbone, et possédant 2 ramifications comportant au moins 6 atomes de carbone.

De manière préférentielle, cet épaississant copolymère est caractérisé en ce qu'il contient, exprimé en poids, de 2 à 25 % et préférentiellement de 5 à 20 % d'au moins un monomère oxyalkylé à insaturation éthylénique et terminé par une chaîne ramifiée hydrophobe et non aromatique comportant de 16 à 20 atomes de carbone, et possédant 2 ramifications comportant au moins 6 atomes de carbone.

De manière encore plus préférentielle, la chaîne ramifiée hydrophobe non aromatique du monomère c) est choisie parmi le 2-hexyl 1-decanyle et le 2-octyl 1-dodecanyle.

Dans une variante également préférentielle, ce copolymère ne contient pas le monomère réticulant d).

Ce copolymère est également caractérisé en ce qu'il est sous sa forme acide ou partiellement ou totalement neutralisé par un ou plusieurs agents de neutralisation disposant d'une fonction neutralisante monovalente tels que ceux choisis dans le groupe constitué par les cations alcalins, en particulier le sodium, le potassium, le lithium, l'ammonium ou les amines primaires, secondaires ou tertiaires aliphatiques et/ou cycliques telles que la stéarylamine, les éthanolamines (mono-, di-, triéthanolamine), la mono et diéthylamine, la cyclohexylamine, la méthylcyclohexylamine, le 2-amino 2-méthyl 1-propanol, la morpholine.

Ce copolymère acrylique hydrosoluble est obtenu par les techniques de copolymérisation bien connues de l'homme du métier, telles que les techniques de copolymérisation radicalaire en solution, en émulsion directe ou inverse, en suspension, ou précipitation dans des solvants appropriés, en présence de systèmes catalytiques et d'agents de transfert connus.

Un autre objet de l'invention concerne aussi le procédé de fabrication de formulations aqueuses éventuellement pigmentées caractérisé en ce que l'on ajoute aux dites formulations ledit copolymère en présence de composés hydrosolubles ou non tels que des sels ou des tensio-actifs ou lors de l'addition des dits composés dans les dites formulations aqueuses.

Un dernier objet de l'invention concerne enfin les formulations aqueuses éventuellement pigmentées contenant ces dits copolymères, telles que les formulations de revêtement comme les peintures en phase aqueuse et notamment les peintures dispersions, les vernis, les sauces de couchage papetières, la cosmétique, la détergence, les formulations textiles et les boues de forage, ou encore les formulations de plâtre, les formulations pour plaques de plâtre, pour liants hydrauliques comme les formulations de mortier ou bien encore les formulations pour céramique et pour cuir.

Les exemples suivants illustrent l'invention sans toutefois en limiter la portée.

### EXEMPLE 1 :

Cet exemple illustre l'utilisation de copolymères selon l'invention dans des formulations de peintures aqueuses, et a pour but de mettre en évidence les propriétés rhéologiques de compatibilité pigmentaire apportées par les copolymères selon l'invention pour des formulations pour peinture mate en phase aqueuse et à dosage constant.

Ainsi, à partir d'une formulation de peinture décoration mate en phase aqueuse à base de liant en dispersion vinyl-acrylique « UCAR™ latex 300 » de Dow dont la formule figure dans le tableau 1, le but a consisté à vérifier dans cette formule la compatibilité pigmentaire (ou compatibilité assurée avec le rajout de concentrés pigmentaires universels) telle qu'influencée par différents épaississants acryliques associatifs dont des épaississants de l'art antérieur dont les groupements hydrophobes sont linéaires et des épaississants selon l'invention, dont les groupements hydrophobes sont ramifiés.

**TABLEAU 1 FORMULE PEINTURE**

| **Composés** | **Rôle** | **Quantité (gramme)** |
|---|---|---|
| | | |
| Eau | Véhicule | 2639 |
| Cellosize™ HEC ER-4400 | Epaississant cellulosique | 13 |
| Tripolyphosphate de potassium | Dispersant | 19,5 |
| Tamol™ 1124 | Dispersant | 65 |
| Triton™ CF-10 | Tensio-actif | 39 |
| Hi-Mar™ DFC-19 | Anti-mousse | 26 |
| Kathon™ LX (1.5%) | Biocide | 19,5 |
| Carbonate de sodium | Agent neutralisant | 46,8 |
| TiPure™ R-900 | Dioxyde de titane | 2340 |
| Huber™ 70C | Argile calcinée | 2906,8 |
| Snowhite™12 | Carbonate de Calcium | 1300 |
| Nyad™ 400 | Wollastonite | 260 |
| Eau | Véhicule | 936 |
| Ucar™ Latex 300 | Liant | 2600 |
| Hi-Mar™ DFC-19 | Anti-mousse | 26 |
| Eau | Diluant final | 2207,4 |

Pour ce faire à chaque essai, on a préparé la formulation de peinture mate précitée dans laquelle est rajouté 0,3 % en poids sec par rapport au poids total de la formule de peinture blanche (mais avant rajout du concentré pigmentaire universel) de l'épaississant à tester puis 36 millilitres d'un concentré pigmentaire universel (de type Colortrend™ M 888 de la société Hüls/ Creanova) ont été rajoutés sur un kilogramme d'une base de peinture blanche préalablement épaissie avec un des épaississants acryliques associatifs.

Deux concentrés pigmentaires universels utilisés ont été choisis dans la gamme Colortrend™ M 888 afin de représenter les principales familles de pigments organiques : le bleu phtalocyanine et le magenta de quinacridone.

A ce stade, une chute ou une augmentation (par rapport à la base blanche) la plus limitée possible de la viscosité Stormer™ exprimée en Krebs Units (KU) traduit une bonne compatibilité avec le concentré pigmentaire universel.

Ce test de la viscosité Stormer™ est utilisé par l'homme du métier comme un test de sélection lui permettant d'éliminer les formulations posant des problèmes de compatibilité avec les concentrés pigmentaires universels.

En effet, l'homme du métier apprécie en particulier que la viscosité Stormer™ qui est une viscosité à moyen gradient de vitesse perceptible par le client (agitation à la spatule ou la brosse, garnissage de la brosse ou du rouleau) soit la moins possible changée par cette addition de concentré pigmentaire universel.

### Essai n° 1 :

Cet essai illustre l'art antérieur et met en oeuvre 0,3 % en poids sec d'un copolymère composé de :
a) 37,2 % d'acide méthacrylique,
b) 52,8 % d'acrylate d'éthyle,
c) 10,0 % du monomère de formule (I) dans lequel
   R représente un radical méthacrylate,
   R'représente une chaîne hydrophobe linéaire à 16 atomes de carbone,
   m = p = 0,
   q = 1,
   n = 25.

### Essai n° 2 :

Cet essai illustre l'invention et met en oeuvre 0,3 % en poids sec d'un copolymère composé de :
a) 37,2 % d'acide méthacrylique,
b) 52,8 % d'acrylate d'éthyle,
c) 10,0 % du monomère de formule (I) dans lequel
   R représente un radical méthacrylate,
   R'représente la chaîne hydrophobe non aromatique à 16 atomes de carbone 2-hexyl 1-decanyle,
   m = p = 0,
   q = 1,
   n = 25.

### Essai n° 3 :

Cet essai illustre l'art antérieur et met en oeuvre 0,3 % en poids sec d'un copolymère composé de :
a) 37,3 % d'acide méthacrylique,
b) 52,6 % d'acrylate d'éthyle,
c) 10,1 % du monomère de formule (I) dans lequel
   R représente un radical méthacrylate,
   R'représente une chaîne hydrophobe linéaire à 16 atomes de carbone,
   m = p = 0,
   q = 1,
   n = 25
et obtenu par mise en oeuvre de 0,05 % en poids de dodécylmercaptan par rapport au poids des monomères.

### Essai n° 4 :

Cet essai illustre l'invention et met en oeuvre 0,3 % en poids sec d'un copolymère composé de :
a) 37,3 % d'acide méthacrylique,
b) 52,6 % d'acrylate d'éthyle,
c) 10, 1% du monomère de formule (I) dans lequel
   R représente un radical méthacrylate,
   R'représente la chaîne hydrophobe non aromatique à 16 atomes de carbone 2 hexyl 1-decanyle,
   m = p = 0,
   q = 1,
   n = 25
   et obtenu par mise en oeuvre de 0,05 % en poids de dodécylmercaptan par rapport au poids des monomères.

### Essai n° 5 :

Cet essai illustre l'art antérieur et met en oeuvre 0,3 % en poids sec d'un copolymère composé de :
a) 43,5 % d'acide méthacrylique,
b) 47,6 % d'acrylate d'éthyle,
c) 8,9 % du monomère de formule (I) dans lequel
   R représente un radical méthacrylate,
   R'représente une chaîne hydrophobe linéaire à 16 atomes de carbone,
   m = p = 0,
   q = 1,
   n = 25
   et obtenu par mise en oeuvre de 0,30 % en poids de dodécylmercaptan par rapport au poids des monomères.

### Essai n° 6 :

Cet essai illustre l'invention et met en oeuvre 0,3 % en poids sec d'un copolymère composé de :
a) 43,5 % d'acide méthacrylique,
b) 47,6 % d'acrylate d'éthyle,
c) 8,9 % du monomère de formule (I) dans lequel
   R représente un radical méthacrylate,
   R'représente la chaîne hydrophobe non aromatique à 16 atomes de carbone 2-hexyl 1-decanyle,
   m = p = 0,
   q = 1,
   n = 25
   et obtenu par mise en oeuvre de 0,30 % en poids de dodécylmercaptan par rapport au poids des monomères.

### Essai n° 7 :

Cet essai illustre l'art antérieur et met en oeuvre 0,3 % en poids sec d'un copolymère composé de :
a) 36,7 % d'acide méthacrylique,
b) 53,1 % d'acrylate d'éthyle,
c) 10,2 % du monomère de formule (I) dans lequel
   R représente un radical méthacrylate,
   R'représente une chaîne hydrophobe linéaire à 20 atomes de carbone,
   m = p = 0,
   q = 1,
   n = 25.

### Essai n° 8 :

Cet essai illustre l'invention et met en oeuvre 0,3 % en poids sec d'un copolymère composé de :
a) 36,7 % d'acide méthacrylique,
b) 53,1 % d'acrylate d'éthyle,
c) 10,2 % du monomère de formule (I) dans lequel
   R représente un radical méthacrylate,
   R'représente la chaîne hydrophobe non aromatique à 20 atomes de carbone 2-octyl 1-dodecanyle,
   m = p = 0,
   q = 1,
   n = 25.

### Essai n° 9 :

Cet essai illustre l'art antérieur et met en oeuvre 0,3 % en poids sec d'un copolymère composé de :
a) 36,7% d'acide méthacrylique,
b) 53,1 % d'acrylate d'éthyle,
c) 10,2 % du monomère de formule (I) dans lequel
   R représente un radical méthacrylate,
   R'représente une chaîne hydrophobe linéaire à 20 atomes de carbone,
   m = p = 0,
   q = 1,
   n = 25
   et obtenu par mise en oeuvre de 0,05 % en poids de dodécylmercaptan par rapport au poids des monomères.

### Essai n° 10 :

Cet essai illustre l'invention et met en oeuvre 0,3 % en poids sec d'un copolymère composé de :
a) 36,7 % d'acide méthacrylique,
b) 53,1 % d'acrylate d'éthyle,
c) 10,2 % du monomère de formule (I) dans lequel
   R représente un radical méthacrylate,
   R'représente la chaîne hydrophobe non aromatique à 20 atomes de carbone 2-octyl 1-dodecanyle, m = p = 0,
   q = 1,
   n = 25
   et obtenu par mise en oeuvre de 0,05 % en poids de dodécylmercaptan par rapport au poids des monomères.

Tous les résultats sont rassemblés dans le tableau 2 suivant.

**TABLEAU 2**

| **Essai n°** | **AA ou INV** | **Viscosité Stormer™ (KU) de la Base blanche** | **Après rajout de colorant bleu** | **Delta KU avec le colorant bleu** | **Après rajout de colorant magenta** | **Delta KU avec le colorant magenta** |
|---|---|---|---|---|---|---|
| **1** | AA | 120 | 125,6 | 5,6 | 131,8 | 11,8 |
| **2** | INV | 95,1 | 97,9 | 2,8 | 103,8 | 8,7 |
| **3** | AA | 127,1 | 132,2 | 5,1 | 133,2 | 6,1 |
| **4** | INV | 97,9 | 101 | 3,1 | 103,5 | 5,6 |
| **5** | AA | 101,8 | 95,9 | 5,9 | 105 | 3,2 |
| **6** | INV | 60,1 | 61,1 | 1 | 60,9 | 0,8 |
| **7** | AA | 107,4 | 119 | 11,6 | 117,8 | 10,4 |
| **8** | INV | 102,7 | 111 | 8,3 | 109,8 | 7,1 |
| **9** | AA | 105,5 | 114,4 | 8,9 | 116,1 | 10,6 |
| **10** | INV | 111,9 | 117,2 | 5,3 | 119,7 | 7,8 |

| | | | | | | |
|---|---|---|---|---|---|---|
| AA = Art antérieur, INV = Invention. | | | | | | |

Dans tous les cas, la variation en valeur absolue de la viscosité Stormer™ des peintures après rajout des colorants est moins importante avec les peintures contenant les épaississants de l'invention par rapport à celle des peintures contenant les épaississants correspondants de l'art antérieur.

### EXEMPLE 2 :

Cet exemple illustre l'utilisation de copolymères selon l'invention dans le domaine des fluides de forage et particulièrement celui des boues synthétiques, et a pour but de mettre en évidence le maintien de l'épaississement conféré par les copolymères selon l'invention pour des formulations à dosage constant en poids sec d'épaississant.

Dans le domaine des fluides de forage et particulièrement des boues synthétiques, l'Homme du métier peut être amené à utiliser une boue constituée uniquement d'un épaississant et d'eau, ceci afin de réduire la quantité de matières non recyclables. Ces boues doivent avoir un pouvoir suspensivant qui bien que diminuant lors du pompage doit rester relativement élevé sous des conditions de stress telles que le passage du fluide dans l'annulaire lors de la remontée des déblais. Les boues traditionnelles à base d'épaississant de l'art antérieur présentent le défaut d'être trop pseudoplastiques, c'est-à-dire perdent la majeure partie de leur viscosité sous cisaillement. Ce phénomène est aisément mesuré en faisant le rapport des viscosités Brookfield™ mesurées à 100T/min sur les viscosités Brookfield™ mesurées à 10T/min Un chiffre faible est l'indication d'une chute importante de viscosité sous cisaillement.

La comparaison des divers polymères s'est faite en réalisant des gels aqueux de ces produits à des concentrations telles que les viscosités Brookfield™ obtenues se situaient entre 1000 mPa.s et 5000 mPa.s à 10 T/min. Les pH de ces gels ont tous été ajustés à 8.

### Essai n° 11 :

Cet essai illustre l'art antérieur et met en oeuvre 1 % en poids sec d'un copolymère composé de :
a) 37,2 % d'acide méthacrylique,
b) 52,8 % d'acrylate d'éthyle,
c) 10,0 % du monomère de formule (I) dans lequel
   R représente un radical méthacrylate,
   R'représente une chaîne hydrophobe linéaire à 16 atomes de carbone,
   m = p = 0,
   q = 1,
   n = 25.

### Essai n° 12 :

Cet essai illustre l'invention et met en oeuvre 1 % en poids sec d'un copolymère composé de :
a) 37,2 % d'acide méthacrylique,
b) 52,8 % d'acrylate d'éthyle,
c) 10,0 % du monomère de formule (I) dans lequel
   R représente un radical méthacrylate,
   R'représente la chaîne hydrophobe non aromatique à 16 atomes de carbone 2-hexyl 1-decanyle,
   m = p = 0,
   q = 1,
   n = 25.

### Essai n° 13 :

Cet essai illustre l'art antérieur et met en oeuvre 1 % en poids sec d'un copolymère composé de :
a) 42,83 % d'acide méthacrylique,
b) 52,97 % d'acrylate d'éthyle,
c) 2,66 % du monomère de formule (I) dans lequel
   R représente un radical méthacrylate,
   R'représente une chaîne hydrophobe linéaire à 16 atomes de carbone,
   m = p = 0,
   q = 1,
   n = 25,
d) 1,54 % de diméthacrylate d'éthylène glycol.

### Essai n° 14 :

Cet essai illustre l'invention et met en oeuvre 1 % en poids sec d'un copolymère composé de :
a) 42,83 % d'acide méthacrylique,
b) 52,97% d'acrylate d'éthyle,
c) 2,66 % du monomère de formule (I) dans lequel
   R représente un radical méthacrylate,
   R'représente la chaîne hydrophobe non aromatique à 16 atomes de carbone 2-hexyl 1-decanyle,
   m = p = 0,
   q = 1,
   n = 25,
d) 1,54 % de diméthacrylate d'éthylène glycol.

### Essai n° 15 :

Cet essai illustre l'art antérieur et met en oeuvre 1 % en poids sec d'un copolymère composé de :
a) 43,5 % d'acide méthacrylique,
b) 47,6 % d'acrylate d'éthyle,
c) 8,9 % du monomère de formule (I) dans lequel
   R représente un radical méthacrylate,
   R'représente une chaîne hydrophobe linéaire à 16 atomes de carbone,
   m = p = 0,
   q = 1,
   n = 25
   et obtenu par mise en oeuvre de 0,30 % en poids de dodécylmercaptan par rapport au poids des monomères.

### Essai n° 16 :

Cet essai illustre l'invention et met en oeuvre 1 % en poids sec d'un copolymère composé de :
a) 43,5 % d'acide méthacrylique,
b) 47,6 % d'acrylate d'éthyle,
c) 8,9 % du monomère de formule (I) dans lequel
   R représente un radical méthacrylate,
   R' représente la chaîne hydrophobe non aromatique à 16 atomes de carbone 2-hexyl 1-decanyle,
   m = p = 0,
   q = 1,
   n = 25
   et obtenu par mise en oeuvre de 0,30 % en poids de dodécylmercaptan par rapport au poids des monomères.

### Essai n° 17 :

Cet essai illustre l'art antérieur et met en oeuvre 1 % en poids sec d'un copolymère composé de :
a) 36,7 % d'acide méthacrylique,
b) 53,1 % d'acrylate d'éthyle,
c) 10,2 % du monomère de formule (I) dans lequel
   R représente un radical méthacrylate,
   R' représente une chaîne hydrophobe linéaire à 20 atomes de carbone,
   m = p = 0,
   q = 1,
   n = 25
   et obtenu par mise en oeuvre de 0,30 % en poids de dodécylmercaptan par rapport au poids des monomères.

### Essai n° 18 :

Cet essai illustre l'invention et met en oeuvre 1 % en poids sec d'un copolymère composé de :
a) 36,7 % d'acide méthacrylique,
b) 53,1 % d'acrylate d'éthyle,
c) 10,2 % du monomère de formule (I) dans lequel
   R représente un radical méthacrylate,
   R'représente la chaîne hydrophobe non aromatique à 20 atomes de carbone 2-hexyl 1-decanyle,
   m = p = 0,
   q = 1,
   n = 25
   et obtenu par mise en oeuvre de 0,30 % en poids de dodécylmercaptan par rapport au poids des monomères.

### Essai n° 19 :

Cet essai illustre l'art antérieur et met en oeuvre 1 % en poids sec d'un copolymère composé de :
a) 42,68 % d'acide méthacrylique,
b) 53,12 % d'acrylate d'éthyle,
c) 2,66 % du monomère de formule (I) dans lequel R représente un radical méthacrylate,
   R'représente une chaîne hydrophobe linéaire à 16 atomes de carbone,
   m = p = 0,
   q = 1,
   n = 25,
d) 1,54 % de diméthacrylate d'éthylène glycol.

### Essai n° 20 :

Cet essai illustre l'invention et met en oeuvre 1 % en poids sec d'un copolymère composé de :
a) 42,68 % d'acide méthacrylique,
b) 53,12 % d'acrylate d'éthyle,
c) 2,66 % du monomère de formule (I) dans lequel
   R représente un radical méthacrylate,
   R'représente la chaîne hydrophobe non aromatique à 16 atomes de carbone 2-hexyl 1-decanyle,
   m = p = 0,
   q = 1,
   n = 25,
d) 1,54 % de diméthacrylate d'éthylène glycol.

### Essai n° 21

Cet essai illustre l'art antérieur et met en oeuvre 1 % en poids sec d'un copolymère composé de :
a) 34,2 % d'acide méthacrylique,
b) 60,4 % d'acrylate d'éthyle,
c) 5,4 % du monomère de formule (I) dans lequel
   R représente un radical méthacrylate,
   R'représente une chaîne hydrophobe linéaire à 20 atomes de carbone,
   m = p = 0,
   q = 1,
   n = 25
   et obtenu par mise en oeuvre de 0,30 % en poids de dodécylmercaptan par rapport au poids des monomères.

### Essai n° 22 :

Cet essai illustre l'invention et met en oeuvre 1 % en poids sec d'un copolymère composé de :
a) 34,2 % d'acide méthacrylique,
b) 60,4 % d'acrylate d'éthyle,
c) 5,4 % du monomère de formule (I) dans lequel
   R représente un radical méthacrylate,
   R'représente la chaîne hydrophobe non aromatique à 20 atomes de carbone 2-octyl 1-dodecanyle,
   m = p = 0,
   q = 1,
   n = 25
   et obtenu par mise en oeuvre de 0,30 % en poids de dodécylmercaptan par rapport au poids des monomères.

Tous les résultats sont rassemblés dans le tableau 3 suivant.

**TABLEAU 3**

| **Essais n°** | **Viscosité Brookfield™ à 20°C, pour 1 % de matière sèche** | | **Rapport viscosité 100 T/min sur viscosité 10 T/min des valeurs pour 1 % de matière sèche** |
|---|---|---|---|
| | **10 T/min** | **100 T/min** | |
| 11 | 9455 | 1709 | 0.181 |
| 12 | 3429 | 2386 | 0.696 |
| 13 | 6000 | 1293 | 0.216 |
| 14 | 4660 | 1097 | 0.235 |
| 15 | 1818 | 1536 | 0.845 |
| 16 | 460 | 452 | 0.983 |
| 17 | 10000 | 1600 | 0.160 |
| 18 | 8226 | 2194 | 0.267 |
| 19 | 7619 | 1270 | 0.167 |
| 20 | 7333 | 1600 | 0.218 |
| 21 | 6500 | 3825 | 0.588 |
| 22 | 2727 | 2848 | 1.044 |

Le tableau 3 ci-dessus illustre parfaitement la nette supériorité des polymères selon l'invention par rapport aux polymères de l'art antérieur, le rapport des viscosités Brookfield™ mesurées à 100 T/min sur les viscosités Brookfield™ mesurées à 10 T/min étant respectivement nettement plus élevé pour les essais illustrant l'invention.

### EXEMPLE 3 :

Cet exemple illustre l'utilisation de copolymères selon l'invention dans le domaine de la détergence et de la cosmétique et a pour but de mettre en évidence le maintien de l'épaississement conféré par les copolymères selon l'invention pour des formulations à dosage constant en poids sec d'épaississant malgré l'ajout de sels.

Tant dans l'application précédente (boues de forage) que dans une application cosmétique ou détergence, la présence de sel est parfois incontournable ou inévitable. En effet, les boues de forage synthétiques peuvent être fabriquées à partir d'eau de mer ou étant fabriquées à partir d'eau douce, peuvent être mises en contact avec des couches géologiques salines. Il est important que la viscosité de la boue résiste à cette contamination afin de garantir son efficacité à remonter des déblais lors du forage. De même, de nombreuses formulations cosmétiques ou détergentes contiennent des sels, utilisés entre autres comme exhausteurs de viscosité vis-à-vis des tensio-actifs, et ce en complément des épaississants ; il est donc important que les dits épaississants résistent à l'ajout de sels. La comparaison des divers polymères s'est faite en réalisant des gels aqueux de ces produits à des concentrations telles que les viscosités Brookfield™ obtenues se situaient entre 1000 et 5000 mPa.s à 10 T/min. Les pH de ces gels ont tous été ajustés à 8. Après mesure des viscosités Brookfield™, des quantités de chlorure de sodium correspondant à 1 et 2 % de la formulation ont été ajoutées et dissoutes. Les viscosités Brookfield™ ont ensuite été mesurées à nouveau.

### Essai n° 23 :

Cet essai illustre l'art antérieur et met en oeuvre 1 % en poids sec du copolymère mis en oeuvre dans l'essai n° 11.

### Essai n° 24 :

Cet essai illustre l'invention et met en oeuvre 1 % en poids sec du copolymère mis en oeuvre dans l'essai n° 12.

### Essai n° 25 :

Cet essai illustre l'art antérieur et met en oeuvre 1 % en poids sec du copolymère mis en oeuvre dans l'essai n° 17.

### Essai n° 26 :

Cet essai illustre l'invention et met en oeuvre 1 % en poids sec du copolymère mis en oeuvre dans l'essai n° 18.

### Essai n° 27 :

Cet essai illustre l'art antérieur et met en oeuvre 1 % en poids sec du copolymère mis en oeuvre dans l'essai n° 21.

### Essai n° 28 :

Cet essai illustre l'invention, et met en oeuvre 1 % en poids sec du copolymère mis en oeuvre dans l'essai n° 22.

Tous les résultats sont rassemblés dans le tableau 4 suivant.

**TABLEAU 4**

| **Essais n°** | **Viscosité Brookfield™ 10 T à 20°C à 1% de matière sèche** | | |
|---|---|---|---|
| | **Sans sel** | **1% NaCl** | **2% NaCl** |
| 23 | 9455 | 727 | 73 |
| 24 | 3429 | 4929 | 3000 |
| 25 | 10000 | 360 | 80 |
| 26 | 8226 | 1129 | 129 |
| 27 | 6500 | 150 | 25 |
| 28 | 2727 | 848 | 121 |

Le tableau 4 ci-dessus illustre parfaitement la supériorité des polymères selon l'invention par rapport aux polymères de l'art antérieur.

### EXEMPLE 4 :

Cet exemple illustre l'utilisation de copolymères selon l'invention dans le domaine de la détergence et de la cosmétique et a pour but de mettre en évidence le développement de l'épaississement conféré par les copolymères selon l'invention pour des formulations à dosage constant en poids sec d'épaississant malgré l'ajout de tensio-actifs.

Les formulations détergentes et cosmétiques contiennent un ou plusieurs tensioactifs ioniques et non ioniques. De tout temps, l'homme du métier a joué sur les propriétés solubilisantes / insolubilisantes de ces couples de tensio-actifs pour épaissir les formulations les contenant. Les polymères épaississants existants sur le marché permettent maintenant de réduire sensiblement les quantités de tensio-actifs, ceux-ci n'étant plus utilisés que pour leurs propriétés détergentes. L'utilisation des polymères de l'invention permet d'améliorer significativement la viscosité des formulations les contenant, par rapport aux polymères épaississants de l'art antérieur. La comparaison des polymères s'est faite par la réalisation de formulations aqueuses contenant 11 % de lauryl alkylsulfate de sodium et 9 % de cocoamidopropyl betaïne. Le pH des formulations a été ajusté à 8 au moyen de triéthanolamine. La viscosité du mélange des tensioactifs sans épaississant est de 20 mPa.s à 10 T/min.

### Essai n° 29 :

Cet essai illustre l'art antérieur et met en oeuvre 1 % en poids sec du copolymère mis en oeuvre dans l'essai n° 11.

### Essai n° 30 :

Cet essai illustre l'invention et met en oeuvre 1 % en poids sec du copolymère mis en oeuvre dans l'essai n° 12.

### Essai n° 31 :

Cet essai illustre l'art antérieur et met en oeuvre 1 % en poids sec du copolymère mis en oeuvre dans l'essai n° 19.

### Essai n° 32 :

Cet essai illustre l'invention et met en oeuvre 1 % en poids sec du copolymère mis en oeuvre dans l'essai n° 20.

### Essai n° 33 :

Cet essai illustre l'art antérieur et met en oeuvre 1 % en poids sec du copolymère mis en oeuvre dans l'essai n° 21.

### Essai n° 34 :

Cet essai illustre l'invention et met en oeuvre 1 % en poids sec du copolymère mis en oeuvre dans l'essai n° 22.

Tous les résultats sont rassemblés dans le tableau 5 suivant.

**TABLEAU 5**

| **Essais n°** | **Viscosité Brookfield™ pour 1% de matière sèche** | |
|---|---|---|
| | **10 T/min** | **100 T/min** |
| 29 | 20000 | 17455 |
| 30 | 27143 | 18143 |
| 31 | 444 | 495 |
| 32 | 867 | 853 |
| 33 | 750 | 744 |
| 34 | 2303 | 2109 |

Le tableau 5 ci-dessus illustre parfaitement la supériorité des polymères selon l'invention par rapport aux polymères de l'art antérieur

### EXEMPLE 5 :

Cet exemple illustre l'utilisation de copolymères selon l'invention dans des sauces de couchage papetières et a pour but de mettre en évidence le maintien de l'épaississement conféré par les copolymères mis en oeuvre selon l'invention pour des formulations à dosage constant en poids sec d'épaississant malgré l'ajout de sels.

Pour ce faire, dans une sauce de couchage composée de 100 parts d'une suspension aqueuse de carbonate de calcium à 78 % de matière sèche commercialisée par la société Omya™ sous le nom Hydrocarb™ 60 et de 10 parts de latex à 50 % de matière sèche commercialisé par la société Dow sous le nom DL 940, on ajoute pour chacun des essais l'épaississant à tester puis on en mesure, après 15 minutes d'agitation la viscosité Brookfield™ à 100 tours par minute à l'aide d'un viscosimètre Brookfield™ muni du module adéquat.

La viscosité Brookfield™ à 100 tours par minute déterminée, on ajoute pour chacun des essais 1,6 parts de chlorure de sodium pulvérulent puis on en mesure, après 15 minutes d'agitation la viscosité Brookfield™ à 100 tours par minute à l'aide d'un viscosimètre Brookfield™ muni du module adéquat.

### Essai n° 35 :

Cet essai illustre l'art antérieur et met en oeuvre le copolymère mis en oeuvre dans l'essai n° 11.

Les viscosités Brookfield™ à 100 tours par minute obtenues sont :
Avant ajout du chlorure de sodium : 3100 mPa.s
Après ajout du chlorure de sodium : 2400 mPa.s

### Essai n° 36 :

Cet essai illustre l'invention et met en oeuvre le copolymère mis en oeuvre dans l'essai n° 12.

Les viscosités Brookfield™ à 100 tours par minute obtenues sont :
Avant ajout du chlorure de sodium : 2700 mPa.s
Après ajout du chlorure de sodium : 3020 mPa.s

La lecture des résultats des deux essais précédents montre la supériorité des polymères de l'invention.

### EXEMPLE 6 :

Cet exemple illustre l'utilisation de copolymères selon l'invention dans le domaine du plâtre.

Pour les essais n° 37 et n° 38, on réalise une formulation de pâte à joint pour plaques de plâtre, respectivement avec un produit de l'art antérieur qui est un copolymère acide méthacrylique/acrylate d'éthyle, et un copolymère composé de :
a) 34,6 % d'acide méthacrylique,
b) 56,1 % d'acrylate d'éthyle,
c) 9,3 % du monomère de formule (I) désigné mono I" et dans lequel mono I" désigne un monomère de formule (I) dans lequel:
   R représente un radical méthacrylate,
   R'représente la chaîne hydrophobe non aromatique à 20 atomes de carbone : 2-octyl 1-dodecanyle,
   m = p = 0,
   q = 1,
   n = 25

La composition des formulations est donnée dans le tableau 6, et lesdites formulations sont réalisées par ajout des constituants, dans l'ordre du tableau, sous agitation dans un mélangeur planétaire.

Après la réalisation du mélange, la viscosité Brookfield™ de la formulation à 10 tours/minute est mesurée aux instants t = 0 et t = 24 heures, selon les méthodes bien connues par l'homme du métier.

**Tableau 6 : composition (en poids) des formulations de plâtre selon l'art antérieur (essai n° 37) et selon l'invention (essai n° 38) et viscosités Brookfield™ à 10 tours/minutes correspondantes mesurées à t = 0 et t = 24 heures**

| **Constituants** | | **Essai n°37 art antérieur** | **Essai n° 38 invention** |
|---|---|---|---|
| Eau ordinaire | | 253.5 g | 253.5 g |
| Antimousse Drewplus™ L475 (commercialisé par DREW™) | | 0.12 g | 0.12 g |
| Biocide Acticide™ MBS (commercialisé par THOR™) | | 023 g | 0.23 g |
| Copolymère acide méthacrylique / acrylate d'éthyle | | 7.7 g | 0 |
| Copolymère essai n° 38 | | 0 | 7.7 g |
| Hydroxyde de sodium (solution à 50 %) | | 1.25 | 1.25 |
| Mica MICA-MU 280 (commercialisé par Comptoir de Minéraux et Matières Premières) | | 30 g | 30g |
| Millwhite Attapulgite Clay (commercialisé par Millwhite Company, Inc.) | | 20 g | 20g |
| Eau ordinaire | | 42 g | 42 g |
| Carbonate de calcium Pulpro 20 (commercialisé par OMYA™) | | 545 g | 545 g |
| Monopropylène Glycol | | 1.5 g | 1.5 g |
| Emulsion acétate de polyvinyle (commercialisé par NACAN™) | | 35 g | 35 g |
| Hydroxyde de sodium 50% | | 0.4 g | 0.4 g |
| Viscosité Brookfield™ (à 10 tours / min) | t = 0 | 101 000 mPa.s | 110 000 mPa.s |
| | t = 24h00 | 95 000 mPa.s | 111 000 mPa.s |

L'utilisation du copolymère selon l'invention permet d'obtenir une viscosité initiale supérieure à celle obtenue avec le polymère de l'art antérieur, et un maintien de cette viscosité dans le temps.

### EXEMPLE 7 :

Cet exemple illustre l'utilisation de copolymères selon l'invention dans le domaine des cosmétiques.

Pour les essais n° 39 et n° 40, on réalise une formulation hydratante pour la peau, dont la composition et l'ordre d'introduction des constituants est donnée dans le tableau 7. Les essais n° 39 et n° 40 mettent en oeuvre respectivement un épaississant selon l'art antérieur qui est un copolymère acide méthacrylique/acrylate d'éthyle, et un copolymère composé de :
a) 36,5 % d'acide méthacrylique,
b) 44,1 % d'acrylate d'éthyle,
c) 10,0 % d'acrylate de butyle,
d) 9,4 % du monomère de formule (I) désigné mono I' et dans lequel mono I' désigne un monomère de formule (I) dans lequel :
   R représente un radical méthacrylate,
   R'représente la chaîne hydrophobe non aromatique à 16 atomes de carbone : 2-hexyl 1-decanyle,
   m = p = 0,
   q = 1,
   n = 25

**Tableau 7 : composition (poids en grammes) des formulations cosmétiques selon les essais n° 39 et n° 40**

| **Noms INCI (International Nomenclature Cosmetic Ingredient)** | | **Noms de marque / Fabricant** |
|---|---|---|
| **Phase A** | | |
| Eau déminéralisée | 57.02 | |
| EDTA sel disodique | 0.05 | |
| Mono propylène glycol | 5 | |

| **Phase B** | | |
|---|---|---|
| Epaississant (selon l'art antérieur : essai n° 39 selon l'invention essai n° 40) | 2.13 | |

| **Phase C** | | |
|---|---|---|
| PEG-6 stéarate, ceteth-20, glyceryl stearate, steareth-20, stéaric acid | 11 | Tefose 2561 /GATTEFOSSE™ |
| Octyldodecyl myristate | 9 | Pelemol 2014 / PHOENIX™ CHEMICAL INC. |
| Cyclomethicone | 6 | Dow Corning 345 Fluid / DOW CORNING™ |
| Huile d'amande douce | 3 | Cropure Almond CRODA™ |

| **Phase D** | | |
|---|---|---|
| Eau déminéralisée | 5 | |

| **Phase E** | | |
|---|---|---|
| TEA 99% | 0.34 | |

| **Phase F** | | |
|---|---|---|
| phenoxyethanol methylparaben butylparaben ethylparaben propylparaben | 1 | Phenonip / CLARIANT™ |

La phase B est ajoutée dans la phase A préalablement homogénéisée.

En parallèle, la phase C est homogénéisée en la portant à 70°C sous agitation.

Les phases A + B homogénéisées sont portées à 70°C et mélangées à la phase C.

Le mélange des phases A+B+C est refroidi sous agitation de 70°C à 60°C, puis sans agitation de 60°C à 30°C.

La phase D est alors ajoutée sous agitation au mélange des phases A + B + C refroidi à 30°C.

Le pH des phases A + B + C + D est alors ajusté à 6.8 avec la phase E.

La phase F est alors ajoutée sous agitation au mélange des phases A + B + C + D + E.

Les viscosités Brookfield™ sont mesurées 24 heures après la préparation à 25°C et sont indiquées dans le tableau 8.

**Tableau 8 : viscosité Brookfield™ des formulations cosmétiques selon l'art antérieur (essai n° 39) et selon l'invention (essai n° 40)**

| **Vitesse de mesure** | **Viscosité Brookfield™ pour l'essai n° 39 (art antérieur)** | **Viscosité Brookfield™ pour l'essai n°40 (invention)** |
|---|---|---|
| 2.5 tours /min | 90000 mPa.s | 156000 mPa.s |
| 5 tours /min | 56000 mPa.s | 102000 mPa.s |
| 10 tours /min | 36000 mPa.s | 70000 mPa.s |
| 20 tours /min | 20750 mPa.s | 46000 mPa.s |
| 50 tours /min | 10900 mPa.s | 29000 mPa.s |
| 100 tours /min | 6900 mPa.s | 17600 mPa.s |

L'utilisation du copolymère selon l'invention permet d'obtenir une viscosité sensiblement supérieure au polymère de l'art antérieur.

Pour les essais n° 41 et n° 42, on réalise une formulation de shampoing, dont la composition et l'ordre d'introduction des constituants est donnée dans le tableau 9 (l'ordre d'introduction n'est pas fondamental mais en respectant la chronologie, la fabrication de la formule s'en trouve facilitée).

Les essais n° 41 (art antérieur) et n° 42 (invention) mettent en oeuvre respectivement un épaississant selon l'art antérieur qui est un copolymère acide méthacrylique/acrylate d'éthyle, et un copolymère composé de :
a) 31,5 % d'acide méthacrylique,
b) 5,0 % d'acide acrylique,
c) 54,1 % d'acrylate d'éthyle,
d) 9,4 % du monomère de formule (I) désigné mono I'.

Une fois le mélange réalisé, la viscosité Brookfield™ est mesurée aux instants t = 0 et t = 24 heures. Ces résultats apparaissent dans le tableau 9.

**Tableau 9 : composition (en poids des constituants) des formulations de shampoing selon l'art antérieur (essai n° 41) et selon l'invention (essai n° 42) et viscosités Brookfield™ correspondantes mesurées à t = 0 et t = 24 heures**

| **Constituants** | | **Essai n° 41 (art antérieur)** | **Essai n°42 (invention)** |
|---|---|---|---|
| Epaississant | | 16 g | 16 g |
| Eau bipermutée | | 109 g | 109 g |
| Sodium Laureth Sulfate Texapon NSO (COGNIST™) | | 60 g | 60 g |
| Hydroxide de sodium 50% | | 1.5 g | 1.5 g |
| Viscosité Brookfield™ àt=0 | 1 tour / min. | 11500 mPa.s | 16000 mPa.s |
| | 10 tours / min. | 2650 mPa.s | 11000 mPa.s |
| | 20 tours / min. | 1775 mPa.s | 7500 mPa.s |
| | 100 tours / min. | 765 mPa.s | 6500 mPa.s |
| Viscosité Brookfield™ à t = 24 heures | 1 tour / min. | 11000 mPa.s | 16000 mPa.s |
| | 10 tours / min. | 2600 mPa.s | 11000 mPa.s |
| | 20 tours / min. | 1750 mPa.s | 9500 mPa.s |
| | 100 tours / min. | 780 mPa.s | 9000 mPa.s |

L'utilisation du copolymère selon l'invention permet d'obtenir une viscosité supérieure au polymère de l'art antérieur.

### EXEMPLE 8 :

Cet exemple illustre l'utilisation de copolymères selon l'invention dans le domaine de la détergence.

Pour les essais n° 43 et n° 44, on réalise une formulation de crème à récurer, dont la composition et l'ordre d'introduction des constituants est donnée dans le tableau 10 (l'ordre d'introduction n'est pas fondamental mais en respectant la chronologie, la fabrication de la formule s'en trouve facilitée).

Les essais n° 43 (art antérieur) et n° 44 (invention), mettent en oeuvre respectivement un épaississant selon l'art antérieur qui est un copolymère acide méthacrylique/acrylate d'éthyle, et un copolymère composé de :
a) 31,5 % d'acide méthacrylique,
b) 5,0 % d'acide itaconique,
c) 54,1 % d'acrylate d'éthyle,
d) 9,4 % du monomère de formule (I) désigné mono I'.

Une fois le mélange réalisé, la viscosité Brookfield™ est mesurée à l'instant t = 0 ; les résultats apparaissent dans le tableau 10.

**Tableau 10 : composition (en poids des constituants) des formulations de crèmes à récurer selon l'art antérieur (essai n° 43) et selon l'invention (essai n° 44) et viscosités Brookfield™ correspondantes mesurées à t = 0**

| **Constituants** | | **Essai n° 43 (art antérieur)** | **Essai n° 44 (invention)** |
|---|---|---|---|
| Monoéthanolamine | | 4g | 4 g |
| Tensioactif Iso tridecanol ethoxylé | | 2g | 2 g |
| Parfum | | 0.4 g | 0.4 g |
| Carbonate de calcium Omyacarb 30AV (OYA™) | | 90 g | 90 g |
| Epaississant | | 6g | 6 g |
| Viscosité Brookfield™ à t = 0 | 1 tour / min. | 10500 mPa.s | 30000 mPa.s |
| | 10 tours / min. | 3100 mPa.s | 25500 mPa.s |
| | 20 tours / min. | 2250 mPa.s | 25000 mPa.s |
| | 100 tours / min. | 1300 mPa.s | 16000 mPa.s |

L'utilisation du copolymère selon l'invention permet d'obtenir une viscosité très supérieure au polymère de l'art antérieur.

### EXEMPLE 9 :

Cet exemple illustre l'utilisation de copolymères selon l'invention dans le domaine des savons liquides.

Les formulations lavantes pour les mains font de plus en plus appel à des savons liquides d'origine naturelle ou synthétique. Contrairement à certains tensioactifs, ces savons ne peuvent souvent pas être épaissis au moyen de chlorure de sodium. Pour des raisons économiques, ils sont souvent dilués et de ce fait perdent toute viscosité. Afin de leur conserver une apparence compatible avec l'utilisation, un agent épaississant doit être utilisé tel que décrit dans les essais suivants.

Pour les essais n° 45 et n° 46, on réalise une formulation de savons liquides, dont la composition et l'ordre d'introduction des constituants est donnée dans le tableau 11 (l'ordre d'introduction n'est pas fondamental mais en respectant la chronologie, la fabrication de la formule s'en trouve facilitée).

Les essais n° 45 (art antérieur) et n° 46 (invention) mettent en oeuvre respectivement un épaississant selon l'art antérieur qui est un copolymère acide méthacrylique/acrylate d'éthyle, et un copolymère composé de :
a) 30,1 % d'acide méthacrylique,
b) 51,4 % d'acrylate d'éthyle,
c) 18,5 % du monomère de formule (I) désigné mono I'.

Une fois le mélange réalisé, le savon est laissé au repos 24 heures puis la viscosité Brookfield™ est mesurée à l'instant t = 24 heures.

**Tableau 11 : composition (en poids des constituants) des formulations de savons liquides selon l'art antérieur (essai n° 45) et selon l'invention (essai n° 46) et viscosités Brookfield™ correspondantes mesurées à t = 24 heures**

| **Constituants** | | **Essai n° 45 (art antérieur)** | **Essai n° 46 (invention)** |
|---|---|---|---|
| Eau ordinaire | | 100 g | 100 g |
| Savon de coprah à 50 % | | 100 g | 100 g |
| Epaississant | | 6 g | 6 g |
| Hydroxyde de potassium 50 % | | qsp pH 9.5 | qsp pH 9.5 |
| Viscosité Brookfield™ | 10 tours / min. | 80 mPa.s | 140 mPa.s |
| | 100 tours / min. | 84 mPa.s | 175 mPa.s |

L'utilisation du copolymère selon l'invention permet d'obtenir une viscosité supérieure au polymère de l'art antérieur.

pour les essais n° 47 et n° 48, on réalise une formulation lavante alcoolique, dont la composition et l'ordre d'introduction des constituants est donnée dans le tableau 12 (l'ordre d'introduction n'est pas fondamental mais en respectant la chronologie, la fabrication de la formule s'en trouve facilitée).

Certaines formulations lavantes pour le sol doivent être efficaces tout en ne laissant aucune trace en l'absence de rinçage. A cet effet, l'homme du métier utilise un mélange d'eau, d'alcool et de tensioactif. Cette formulation doit être légèrement épaissie afin de pouvoir être utilisée avec facilité.

Les essais n° 47 (invention) et n° 48 (référence) mettent en oeuvre respectivement un copolymère composé de :
a) 32,1 % d'acide méthacrylique,
b) 55,3 % d'acrylate d'éthyle,
c) 12,6 % du monomère de formule (I) désigné mono I',
   et aucun épaississant.

Une fois le mélange réalisé, la viscosité Brookfield™ est mesurée à l'instant t = 0 ; les résultats apparaissent dans le tableau 12.

**Tableau 12 : composition (en poids des constituants) des formulations lavantes alcooliques selon la référence (essai n° 48) et selon l'invention (essai n° 47) et viscosités Brookfield™ correspondantes mesurées à t = 0**

| **Constituants** | **Essai n° 47 (invention)** | **Essai n° 48 (référence)** |
|---|---|---|
| Ethanol dénaturé | 45 g | 45 g |
| Eau bipermutée | 105 g | 105 g |
| Epaississant | 5 g | 0 g |
| Polyglycol d'alcool gras éthoxylé (8 OE) ZUZOLAT 1008/25 (ZSCHIMMER & SCHWARZ™) | 2 g | 2 g |
| Triéthanolamine | qsp pH 8.0 | qsp pH 8.0 |
| Viscosité Brookfield™ (10 tours / min.) | 140 mPa.s | 20 mPa.s |

L'utilisation du copolymère selon l'invention permet d'épaissir sensiblement la formulation selon l'invention.

### EXEMPLE 10 :

Cet exemple illustre l'utilisation de copolymères selon l'invention dans le domaine du textile.

Pour les essais n° 49 et n° 50, on réalise une pâte d'impression textile, dont la composition et l'ordre d'introduction des constituants est donnée dans le tableau 13 (l'ordre d'introduction n'est pas fondamental mais en respectant la chronologie, la fabrication de la formule s'en trouve facilitée).

Les essais n° 49 (invention) et n° 50 (référence) mettent en oeuvre respectivement un épaississant qui est un copolymère composé de :
a) 34,5 % d'acide méthacrylique,
b) 56,1 % d'acrylate d'éthyle,
c) 9,4 % du monomère de formule (I) désigné mono I',
   et aucun épaississant.

Une fois le mélange réalisé, la viscosité Brookfield™ est mesurée à l'instant t = 0 ; les résultats apparaissent dans le tableau 13.

**Tableau 13 : composition (en poids des constituants) des pâtes d'impression textile selon la référence (essai n° 50) et selon l'invention (essai n° 49) et viscosités Brookfield™ correspondantes mesurées à t = 0**

| **Constituants** | | **Essai n° 49 (invention)** | **Essai n° 50 (référence)** |
|---|---|---|---|
| Eau | | 212.2 g | 212.2 g |
| Antimousse Bubblex 250 (COGNIS™) | | 1 g | 1 g |
| Emulsion acrylique pour pâte d'impression textile | | 30.8 g | 30.8 g |
| Ammoniaque 28% | | qsp pH 8 - 8.5 | qsp pH8-8.5 |
| Epaississant | | 6 g | 0 g |
| Viscosité Brookfield™ à t = 0 | 1 tour / min. | 1000 mPa.s | 20 mPa.s |
| | 10 tours / min. | 300 mPa.s | 20 mPa.s |
| | 20 tours / min. | 250 mPa.s | 20 mPa.s |
| | 100 tours / min. | 200 mPa.s | 20 mPa.s |

L'utilisation du copolymère selon l'invention permet d'épaissir notablement la formulation.

### EXEMPLE 11 :

Cet exemple illustre l'utilisation de copolymères selon l'invention dans le domaine de la céramique.

Dans cet exemple, on réalise une formulation appelée véhicule d'émaillage car elle est utilisée comme vecteur pour l'émaillage des céramiques telles que, et de manière non limitative, les carreaux et les pièces sanitaires. Cette formulation sera utilisée par l'homme du métier comme liquide dans lequel des émaux en poudre seront dispersés puis éventuellement broyés. L'ajout d'un épaississant permet d'obtenir une viscosité finale correcte permettant à la fois une application aisée et une stabilisation de la dispersion d'émail au stockage.

Pour les essais n° 51 et n° 52, on réalise une formulation de véhicule d'émaillage, dont la composition et l'ordre d'introduction des constituants est donnée dans le tableau 14 (l'ordre d'introduction n'est pas fondamental mais en respectant la chronologie, la fabrication de la formule s'en trouve facilitée).

Les essais n° 51 (art antérieur) et n° 52 (invention) mettent en oeuvre respectivement un épaississant de l'art antérieur qui est un copolymère acide méthacrylique / acrylate d'éthyle, et un copolymère composé de :
a) 36,5 % d'acide méthacrylique,
b) 44,1 % d'acrylate d'éthyle,
c) 10,0 % d'acétate de vinyle,
d) 9,4 % du monomère de formule (I) désigné mono I'.

Une fois le mélange réalisé, la viscosité de la formulation est mesurée au moyen d'une coupe Ford munie d'un orifice de 4 mm, selon les méthodes bien connues de l'homme du métier.

La composition des formulations ainsi que les valeurs de viscosité obtenues sont indiquées dans le tableau 14.

**Tableau 14 : composition (en poids des constituants) pour les essais n° 51 (art antérieur) et n° 52 (invention) et viscosités coupe Ford obtenues**

| **Constituants** | **Essai n° 51 (art antérieur)** | **Essai n° 52 (invention)** |
|---|---|---|
| Eau ordinaire | 101.1 g | 101.1 g |
| Antimousse Bubblex™ 250 (COGNIS™) | 1.2 g | 1.2 g |
| Epaississant | 5 g | 5 g |
| Hydroxyde de sodium 50 % | 0.5 g | 0.5 g |
| Monopropylene glycol | 90 g | 90 g |
| Dispersant Coatex™ DV 204 (COATEX™) | 2 g | 2 g |
| Viscosité Coupe Ford diamètre 4 | 16 secondes | 20 secondes |

L'utilisation du copolymère selon l'invention permet d'obtenir une viscosité supérieure à celle résultant de l'utilisation du polymère selon l'art antérieur.

Pour les essais n° 53 et n° 54, on réalise des formulations de barbotine qui sont utilisées dans le domaine de la céramique.

La fabrication de pièces et matériaux céramique fait appel à l'utilisation de dispersions d'argiles et de minéraux divers broyés en phase aqueuse. Dans le cas de certaines pièces, la dispersion broyée de ces minéraux, appelée barbotine peut être conservée sous forme liquide afin d'être utilisée par moulage ou coulage. Si certaines applications nécessitent une barbotine de faible viscosité, d'autres imposent une viscosité plus élevée afin soit de maintenir les particules de minéraux en suspension ou de donner à la barbotine des caractéristiques de viscosité permettant son application par coulage en moule ou en bande. A cet effet on peut utiliser un épaississant en phase aqueuse tel que décrit dans les essais suivants.

Pour les essais n° 53 et n° 54, on réalise une formulation de barbotine, dont la composition et l'ordre d'introduction des constituants est donnée dans le tableau 15 (l'ordre d'introduction n'est pas fondamental mais en respectant la chronologie, la fabrication de la formule s'en trouve facilitée).

Les essais n° 53 (invention) et n° 54 (référence) mettent en oeuvre respectivement un copolymère composé de :
a) 36,5 % d'acide méthacrylique,
b) 44,1 % d'acrylate d'éthyle,
c) 10,0 % d'acrylamide,
d) 9,4 % du monomère de formule (I) désigné mono I',
   et aucun épaississant.

Une fois le mélange réalisé, la viscosité de la formulation est mesurée au moyen d'un viscosimètre Brookfield™, à la vitesse de 10 tours/minute.

Les compositions des barbotines ainsi que lesdites viscosités Brookfield™ sont indiquées dans le tableau 15.

**Tableau 15 : composition (en poids des constituants) des barbotines pour les essais n° 53 (invention) et n° 54 (référence) et viscosités Brookfield™ obtenues**

| **Constituants** | **Essai n° 53 (invention)** | **Essai n° 54 (référence)** |
|---|---|---|
| Eau ordinaire | 204.2 g | 204.2 g |
| Dispersant acrylique Coatex™ GX CE (COATEX™) | 3.57 g | 3.57 g |
| Mélange d'argiles brovées | 500 g | |
| Dispersion sous agitation pendant 30 minutes | | |
| Epaississant | 0.71 g | 0 g |
| Agitation puis ajustement du pH jusqu'à une valeur de 8.5 au moyen d'hydroxyde de sodium 50 % | | |
| Viscosité Brookfield™ 10 T/min | 3600 mPa.s | 900 mPa.s |

L'utilisation du copolymère selon l'invention permet d'épaissir notablement la barbotine.

### EXEMPLE 12 :

Cet exemple illustre l'utilisation de copolymères selon l'invention dans le domaine du cuir.

Les articles en peau (cuir) doivent souvent être recouvert d'une pellicule protectrice afin de garantir une apparence uniforme et / ou une résistance à l'eau. Cette protection peut être réalisée par l'application d'un vernis en phase aqueuse. La formulation ci-dessous exemplifie l'utilisation du polymère de l'invention afin d'épaissir une formulation à base d'une émulsion acrylique en phase aqueuse, épaississement nécessaire à une application aisée par tout moyen connu de l'homme du métier. En effet, cette formulation non épaissie a une viscosité très faible se traduisant par des coulures lors de l'application du vernis si ce dernier n'est pas épaissi.

Pour les essais n° 55 et n° 56, on réalise une formulation de vernissage destinée aux cuirs, dont la composition et l'ordre d'introduction des constituants est donnée dans le tableau 16 (l'ordre d'introduction n'est pas fondamental mais en respectant la chronologie, la fabrication de la formule s'en trouve facilitée).

Les essais n° 55 (art antérieur) et n° 56 (invention) mettent en oeuvre respectivement un épaississant de l'art antérieur qui est un copolymère acide méthacrylique / acrylate d'éthyle, et le copolymère mis en oeuvre dans l'essai n° 42.

Une fois la formulation réalisée, la viscosité Brookfield™ est mesurée, à 10 et à 100 tours/minute, afin de vérifier l'effet épaississant des produits.

Les compositions des formulations, ainsi que les viscosités Brookfield™ correspondantes sont indiquées dans le tableau 16.

**Tableau 16 : composition (en poids des constituants) des formulations de vernissage pour cuir pour les essais n° 55 (art antérieur) et n° 56 (invention) et viscosités Brookfield™ obtenues**

| **Constituants** | | **Essai n° 55 (art antérieur)** | **Essai n° 56 (invention)** |
|---|---|---|---|
| Emulsion acrylique en phase aqueuse (vernis) Acronal™ 18D (BASF™) | | 180 g | 180 g |
| Eau | | 62 g | 62 g |
| Triéthanolamine | | qsp pH 9 - 9.5 | qsp pH 9 - 9.5 |
| Epaississant | | 2 g | 2 g |
| Triéthanolamine | | qsp pH 8 - 8.5 | qsp pH 8 - 8.5 |
| Viscosité Brookfield™ | 10 tours / minute | 4100 mPa.s | 5500 mPa.s |
| | 100 tours / minute | 890 mPa.s | 1200 mPa.s |

L'utilisation du copolymère selon l'invention permet d'obtenir une viscosité supérieure au polymère de l'art antérieur.

Pour les essais n° 57 et n° 58, on réalise une formulation du type émulsion grasse pour le cuir.

De très nombreuses émulsions grasses sont utilisées lors du traitement des cuirs et peaux afin d'apporter au produit fini des caractéristiques entre autre mais de manière non limitative de souplesse et résistance à l'eau. Ces émulsions constituées d'huiles d'origine animale, végétale ou synthétique peuvent être transportées sur de grandes distances ou stockées pour des périodes prolongées. Se pose alors le problème de leur stabilité, problème qui peut être partiellement résolu en augmentant leur teneur en matière active. Toutefois, cette augmentation doit souvent être importante afin d'obtenir une émulsion stable et se traduit généralement par une viscosité très élevée rendant la manipulation du produit difficile. L'utilisation d'un polymère épaississant permet de pallier à cet inconvénient en apportant une viscosité importante au repos mais diminuant rapidement lors de la manipulation de l'émulsion, permettant ainsi l'obtention d'un produit stable mais facilement manipulable. Les essais ci-dessous mettent en exergue la propriété du polymère de l'invention à remplir cette fonction.

Pour les essais n° 57 et n° 58, on réalise une formulation du type émulsion grasse destinée aux cuirs, dont la composition et l'ordre d'introduction des constituants est donnée dans le tableau 17 (l'ordre d'introduction n'est pas fondamental mais en respectant la chronologie, la fabrication de la formule s'en trouve facilitée).

Les essais n° 57 (art antérieur) et n° 58 (invention) mettent en oeuvre respectivement un épaississant de l'art antérieur qui est un copolymère acide méthacrylique / acrylate d'éthyle, et le copolymère mis en oeuvre dans l'essai n° 47.

Une fois la formulation réalisée, la viscosité Brookfield™ à 10 et à 100 tours / minute est mesurée afin de vérifier l'effet épaississant des produits.

La composition des formulations ainsi que le rapport des viscosités Brookfield™ à 10 et 100 tours/minute sont indiquées dans le tableau 17.

**Tableau 17 : composition (en poids des constituants) des formulations du type émulsion grasse pour le cuir pour les essais n° 57 (art antérieur) et n° 58 (invention) et viscosités Brookfield™ obtenues**

| **Constituants** | | **Essai n° 57 (art antérieur)** | **Essai n° 58 (invention)** |
|---|---|---|---|
| Emulsion d'huiles végétales | | 60 g | 60 g |
| Eau ordinaire | | 60 g | 60 g |
| Epaississant | | 2.4 g | 2.4 g |
| Ammoniaque 28% | | qsp pH 7.3 | qsp pH 7.3 |
| Viscosité Brookfield™ | 10 T/min | 800 mPa.s | 11300 mPa.s |
| | 100 T/min | 225 mPa.s | 2650 mPa.s |
| Rapport des viscosités Brookfield™ à 10 et 100 tours / minute | | 3.55 | 4.26 |

Le copolymère de l'invention permet d'obtenir une viscosité Brookfield™ très supérieure au polymère de l'art antérieur avec de plus un rapport entre les viscosités Brookfield™ à 10 et 100 tours/minute supérieur, se traduisant à viscosité Brookfield™ à 10 tours/minute identique par une plus grande stabilité au repos assortie d'une manipulation plus aisée de part une viscosité Brookfield™ à 100 tours/minute plus faible.

### EXEMPLE 13 :

Cet exemple illustre l'utilisation de copolymères selon l'invention dans le domaine du forage.

Lors du forage de puits destinés à la recherche de pétrole ou d'autre matières telles que l'eau ou le gaz, l'homme du métier va devoir consolider les parois du puits lorsque celui-ci doit entrer en exploitation ou au cours du forage si le besoin s'en fait sentir. A cet effet, il est couramment employé des dispersions aqueuses de ciment dont la composition la plus simple est à base d'eau, d'épaississant, de dispersant et de ciment. D'autres produits peuvent être utilisés tels que des alourdissants (baryte, hématite) ou d'autres additifs tels que des agents moussants ou réducteurs de filtrat. La formulation ci-dessous n'est donc en aucun cas limitative.

Pour les essais n° 59 et n° 60, on réalise une formulation de dispersion de ciment pour utilisation dans les puits de forage, dont la composition et l'ordre d'introduction des constituants est donnée dans le tableau 18 (l'ordre d'introduction n'est pas fondamental mais en respectant la chronologie, la fabrication de la formule s'en trouve facilitée).

Les essais n° 59 (invention) et n° 60 (référence) mettent en oeuvre respectivement un copolymère composé de :
a) 8,4 % d'acide méthacrylique,
b) 82,2 % d'acrylate d'éthyle,
c) 9,4 % du monomère de formule (I) désigné mono I',
   et aucun épaississant.

Une fois la dispersion réalisée, la viscosité est mesurée au moyen d'une Coupe Ford™ d'un diamètre de 4 mm afin de vérifier l'effet épaississant du polymère de l'invention.

Les compositions des formulations ainsi que les viscosités obtenues sont indiquées dans le tableau 18.

**Tableau 18 : composition (en poids des constituants) des formulations du type dispersion de ciment pour les puits de forage pour les essais n° 59 (invention) et n° 60 (référence) et viscosités Coupe Ford™ obtenues**

| **Constituants** | **Essai n° 59 (invention)** | **Essai n° 60 (référence)** |
|---|---|---|
| Ciment CEM I 42.5 R | 400 g | 400 g |
| Eau ordinaire | 300 g | 300 g |
| Epaississant | 1 g | 0 g |
| Viscosité Coupe Ford diamètre 4 | 14 secondes | 10 secondes |

Une faible dose du copolymère de l'invention permet d'épaissir la dispersion.

Pour les essais n° 61 à n° 65, on réalise des boues bentonitiques à base d'eau douce ou d'eau de mer.

Lors du forage d'un puit dans le but de rechercher du pétrole, du gaz ou de l'eau, l'homme du métier utilise entre autre un fluide de forage dont le but est de refroidir le trépan et remonter les déblais. Ce fluide de forage est très souvent à base d'eau, douce ou saline et contient diverses charges minérales ayant pour but de lui donner une certaine consistance et une certaine densité. Cette boue doit avoir des caractéristiques de viscosité très bien contrôlées afin de pouvoir aisément remonter les déblais tout en restant pompable. L'utilisation d'un polymère épaississant permet d'ajuster finement cette viscosité pour répondre au cahier des charges défini sur le lieu de forage et dépendant du type de formation rocheuse traversée. Les boues de forage ci-dessous sont réalisées par mélange sous agitation à haute vitesse dans un mélangeur Hamilton Beach™ des composés suivants. L'ordre d'introduction n'est pas fondamental mais en respectant la chronologie et les temps d'ajout, la fabrication de la formule s'en trouve facilitée.

L'eau de mer synthétique est réalisée par dissolution dans un volume final de 1 litre des sels suivants :

| | |
|---|---|
| Chlorure de sodium | 44.05 g. |
| Chlorure de potassium | 0.67 g. |
| Chlorure de calcium dihydraté | 1.36 g. |
| Chlorure de magnésium hexahydraté | 4.66 g. |
| Sulfate de magnésium heptahydraté | 6.29 g. |
| Hydrogénocarbonate de sodium | 0.18 g. |

Après la préparation des boues, leurs caractéristiques rhéologiques sont mesurées au moyen d'un rhéomètre Fann™. L'utilisation de polymère épaississant vise à augmenter la valeur des viscosités mesurées.

Les essais n° 61 (référence), 62 (art antérieur) et 63 (invention) sont relatifs à des formulations en eau douce, et mettent respectivement en oeuvre : aucun épaississant, un polymère de l'art antérieur qui est un copolymère acide méthacrylique / acrylate d'éthyle, et un copolymère composé de :
a) 35,3 % d'acide méthacrylique,
b) 52,7 % d'acrylate d'éthyle,
c) 12,0 % du monomère de formule (I) désigné mono I" composé de :
   Les essais n° 64 (référence) et n° 65 (invention) sont relatifs à des formulations en eau de mer, et mettent respectivement en oeuvre : aucun épaississant, et un copolymère composé de :
      a) 20,8 % d'acide méthacrylique,
      b) 70,0 % d'acrylate d'éthyle,
      c) 9,2 % du monomère de formule (I) désigné mono I".

Les compositions et les viscosités correspondantes sont indiquées dans le tableau 19.

**Tableau 19 : composition des formulations de boues bentonitiques en eau douce et en eau de mer, et viscosités Fann™ correspondantes**

| **Constituants** | | **Essai n° 61 (référence)** | **Essai n° 62 (art antérieur)** | **Essai n° 63 (invention)** | **Essai n° 64 (référence)** | **Essai n° 65 (invention)** |
|---|---|---|---|---|---|---|
| Eau ordinaire | | 375.5 g | 375.5 g | 375.5 g | 0 g | 0 g |
| Eau de mer synthétique | | 0 g | 0 g | 0 g | 334.8 g | 334.8 g |
| Argile bentonitique Zeogel™ (BAROID™) | | 7.66 g | 7.66 g | 7.66 g | 9g | 9g |
| Agitation pendant 10 minutes | | | | | | |
| Argile bentonitique Aquagel™ (BAROID™) | | 6.13 g | 6.13 g | 6.13 g | 7.24 g | 7.24 g |
| Agitation pendant 10 minutes | | | | | | |
| Sulfate de baryum (Baryte) | | 207 g | 207 g | 207 g | 244 g | 244 g |
| Agitation pendant 15 minutes | | | | | | |
| Réducteur de filtrat ThermaCheck™ (BAROID™) | | 3.83 g | 3.83 g | 3.83 g | 4.52 g | 4.52 g |
| Agitation pendant 10 minutes puis ajustement du pH a 10.5 avec de l'hydroxyde sodium 50% | | | | | | |
| Epaississant | | 0 g | 2.4 g | 2.4 g | 0 g | 0.6 g |
| Agitation pendant 10 minutes puis mesure des viscosités au viscosimètre Fann™ | | | | | | |
| Viscosité Fann™ | 600 T/min | 120 | 195 | 205 | 60 | 114 |
| | 300 T/min | 84 | 138 | 147 | 39 | 81 |
| | 200 T/min | 67 | 111 | 120 | 31 | 67 |
| | 100 T/min | 46 | 75 | 84 | 23 | 52 |
| | 6T/min | 12 | 19 | 25 | 10 | 30 |
| | 3 T/min | 10 | 16 | 22 | 10 | 29 |
| | 0.3 T/min après 10 minutes de repos | 24 | 58 | 71 | 21 | 47 |

Le copolymère de l'invention permet d'obtenir une viscosité supérieure au polymère de l'art antérieur. Ceci est particulièrement visible sur la mesure à 0.3 T/min après 10 minutes de repos qui représente la capacité de la boue à maintenir en suspension les déblais lors d'un arrêt des pompes de circulation de la boue. Une valeur élevée de cette mesure est nécessaire pour éviter toute sédimentation des déblais au fond du puit, sédimentation qui entraînerait un blocage du forage.

### EXEMPLE 14 :

Cet exemple illustre l'utilisation de copolymères selon l'invention dans le domaine des liants hydrauliques.

Lors de l'utilisation de liants hydrauliques tels que le ciment, l'homme du métier peut être amené à fabriquer une composition cimentaire dont la viscosité doit être augmentée afin de favoriser la tenue du mélange lors de l'application ou lors d'un démoulage précoce. Cette application n'est bien entendu nullement limitative et le polymère de l'invention pourra être utilisé à chaque fois qu'un épaississement est nécessaire dans une composition cimentaire ou de liant hydraulique, pour quelque application que ce soit.

Pour les essais n° 66 et n° 67, on réalise une formulation de mortier, dans un mélangeur à mortier, dont la composition et l'ordre d'introduction des constituants est donnée dans le tableau 20 (l'ordre d'introduction n'est pas fondamental mais en respectant la chronologie, la fabrication de la formule s'en trouve facilitée).

Les essais n° 66 (invention) et n° 67 (référence) mettent respectivement en oeuvre un copolymère composé de :
a) 20,0 % d'acide méthacrylique,
b) 70,6 % d'acrylate d'éthyle,
c) 9,4 % du monomère de formule (I) désigné mono I',
   et aucun épaississant.

Une fois la formule fabriquée, la viscosité est mesurée au moyen d'une table à choc afin de vérifier l'effet épaississant du polymère de l'invention. Cet épaississement se traduit par un étalement réduit de la galette de mortier sur la table à choc.

La composition des formulations ainsi que les valeurs des étalements correspondant apparaissent dans le tableau 20.

**Tableau 20 : composition des mortiers, et étalements correspondants**

| **Constituants** | **Essai n° 66 (invention)** | **Essai n° 67 (référence)** |
|---|---|---|
| Eau ordinaire | 300 g | 300 g |
| Epaississant | 3 g | 0 g |
| Hydroxyde de lithium | 1 g | 1 g |
| Ciment CEM I 42.5 R | 450 g | 450 g |
| | Agitation petite vitesse pendant 30 secondes | |
| Sable normalisé (EN 196-1) Ajouté en 30 secondes | 1590 g | 1590 g |
| | Agitation grande vitesse pendant 30 secondes | |
| | Repos pendant 90 secondes | |
| | Agitation grande vitesse pendant 60 secondes | |
| Etalement à la table à choc | 19.6 cm | 24 cm |

Le copolymère de l'invention permet de réduire sensiblement l'étalement du mortier, ce qui traduit une augmentation de viscosité.

### EXEMPLE 15:

Cet exemple illustre l'utilisation de copolymères selon l'invention dans le domaine des plâtres.

Le plâtre de Paris (sulfate de calcium, hémihydrate) est très souvent utilisé dans des compositions liantes, collantes ou de jointoiement pour la construction. II peut être associé à d'autres charges minérales ou non. Certaines utilisations imposent à la dispersion d'avoir une certaine viscosité afin de faciliter sa mise en oeuvre par l'homme du métier.

La formulation suivante est composée de plâtre de Paris et de carbonate de calcium. Sa viscosité est contrôlée au moyen d'un anneau de Schmidt™ d'un diamètre intérieur de 6 cm. Cet anneau est posé sur une plaque de verre, rempli de la formulation et soulevé d'un mouvement rapide et régulier. La dispersion de plâtre s'étale alors en une galette d'un diamètre proportionnel à sa viscosité. Dans ce cas, on recherche un étalement nul, la dispersion bien qu'aisément manipulable devant rester immobile. Utilisé par exemple en jointoiement, cette formulation restera en place sans se déformer après son application.

Pour les essais n° 68 et n° 69, on réalise une formulation de plâtre, dont la composition et l'ordre d'introduction des constituants est donnée dans le tableau 21 (l'ordre d'introduction n'est pas fondamental mais en respectant la chronologie, la fabrication de la formule s'en trouve facilitée).

Les essais n° 68 (art antérieur) et n° 69 (invention) mettent respectivement en oeuvre un épaississant de l'art antérieur qui est un copolymère de l'acide acrylique et de l'acrylate d'éthyle, et un copolymère constitué de :
a) 36,5 % d'acide méthacrylique,
b) 44,1 % d'acrylate d'éthyle,
c) 10,0 % de méthacrylamide,
d) 9,4 % du monomère de formule (I) désigné mono I'.

**Tableau 21 : composition des plâtres, et étalements correspondants**

| **Constituants** | **Essai n° 68 (art antérieur)** | **Essai n° 69 (invention)** |
|---|---|---|
| Sulfate de calcium, hémihydrate Plâtre de Paris | 140 g | 140 g |
| Carbonate de calcium Omyacarb™ 30 AV (OMYA™) | 40 g | 40 g |
| Eau ordinaire | 120 g | 120 g |
| Epaississant | 0.2 g | 0.2 g |
| 2-amino 2-méthyl 1-propanol AMP 95 (ANGUS™) | 0.2 g | 0.2 g |
| Etalement à l'anneau de Schmidt™ | 8 cm | 6 cm |

Le copolymère de l'invention permet de maintenir la formulation en place alors que le polymère de l'art antérieur laisse une déformation se produire.

### EXEMPLE 16 :

Cet exemple concerne l'utilisation des polymères selon l'invention dans le domaine des peintures.

Pour les essais n° 70 à n° 84, on réalise des formulations de peinture, à partir d'une base satinée, et par ajout d'un polymère selon l'art antérieur ou par ajout d'un polymère selon l'invention.

La composition de la base satinée est la suivante (poids en grammes) :

| | |
|---|---|
| Prolylène glycol: | 40 |
| Eau : | 134 |
| Coatex™ BR3 (COATEX™) : | 5 |
| Mergal™ K6N (RIEDEL DE HAEN™) : | 2 |
| Nopco™ NDW (HENKEL™) : | 1 |
| TiO2 RHD2 : | 200 |
| Hydrocarb™ (OMYA™) : | 150 |
| Acronal™ 290 D (BASF™): | 420 |
| Butyldiglycol : | 30 |
| Ammoniaque (31 %) : | 3 |

L'essai n° 70 illustre l'art antérieur et met en oeuvre 985 grammes de ladite base satinée, ainsi que 14,1 grammes d'un polymère de l'art antérieur qui est un copolymère de l'acide méthacrylique et de l'acrylate d'éthyle, et 0,9 gramme d'eau.

Les essais n° 71 à n° 84 illustrent l'invention et mettent eu oeuvre 985 grammes de ladite base satinée, et 15 grammes de différents copolymères, selon l'invention, identifiés par le numéro des essais où ils ont déjà été mis en oeuvre.

On réalise alors 2 types de tests sur ces différentes formulations de peinture :
- un test de compatibilité pigmentaire, qui consiste à mesurer la viscosité Brookfield™ à 10 et 100 tours/minute sur chaque formulation, puis après ajout de 5 % en poids par rapport au poids total de la formulation, d'un pigment noir qui est le Colanyl N130 (CLARIANT™); on calcule alors la variation de viscosité, exprimée en pourcentage d'évolution par rapport à la viscosité initiale, et notée Δ₁₀^{comp} et Δ₁₀₀^{comp} selon qu'elle se réfère à une mesure effectuée à 10 et 100 tours/minute; moins cette variation est importante, plus la compatibilité pigmentaire s'avère être bonne ;
- un test de stabilité à l'étuve, qui consiste à mesurer la viscosité Brookfield™ à 10 et 100 tours/minute sur chaque formulation, puis après un séjour de 10 jours dans une étuve à 50 °C ; on calcule alors la variation de viscosité, exprimée en pourcentage d'évolution par rapport à la viscosité initiale, et notée Δ₁₀^{stab} et Δ₁₀₀^{stab} selon qu'elle se réfère à une mesure effectuée à 10 et 100 tours/minute ; plus cette variation est proche de zéro, meilleure s'avère la stabilité;

Pour les essais n° 70 à n° 84, les polymères utilisés, ainsi que les valeurs de Δ₁₀^{comp} et Δ₁₀₀^{comp} obtenues pour les tests de compatibilité pigmentaire, et les valeurs de Δ₁₀^{stab} et Δ₁₀₀^{stab} obtenues pour les tests de stabilité à la température, sont indiquées dans le tableau 22.

| **Essai n°** | **70** | **71** | **72** | **73** | **74** | **75** | **76** | **77** | **78** | **79** | **80** | **81** | **82** | **83** | **84** |
|---|---|---|---|---|---|---|---|---|---|---|---|---|---|---|---|
| **Polymère de l'essai n°** | * | 42 | 44 | 49 | 40 | 52 | 53 | 59 | 69 | 66 | 47 | 46 | 65 | 38 | 63 |
| **Δ₁₀^{comp}** | 41 | 27 | 40 | 16 | 29 | 15 | 33 | 40 | 21 | 28 | 30 | 22 | 9 | 8 | 5 |
| **Δ₁₀₀^{comp}** | 41 | 27 | 33 | 13 | 26 | 18 | 39 | 26 | 21 | 22 | 30 | 26 | 7 | 14 | 18 |
| **Δ₁₀^{stab}** | -35 | -32 | 17 | -9 | -30 | -17 | -10 | -30 | -7 | -25 | -32 | -12 | -19 | -6 | 0 |
| **-Δ₁₀₀^{stab} -Δ₁₀₀** | -36 | -23 | 12 | 2 | -28 | -7 | -5 | -32 | -2 | -18 | -29 | 2 | -13 | 4 | 29 |

| | | | | | | | | | | | | | | | |
|---|---|---|---|---|---|---|---|---|---|---|---|---|---|---|---|
| * copolymére de l'acide méthacrylique et de l'acrylate d'éthyle | | | | | | | | | | | | | | | |

**Tableau 22** : polymères utilisés dans les essais n° 70 (art antérieur) et n° 71 à 84 (invention - les polymères sont ici repérés par les numéros des essais où ils ont été précédemment mis en oeuvre), ainsi que valeurs de Δ₁₀^{comp} et Δ₁₀₀^{comp} (en % d'augmentation relatif) obtenues pour les tests de compatibilité pigmentaire, et valeurs de Δ₁₀^{stab} et Δ₁₀₀^{stab} (en % d'augmentation relatif) obtenues pour les tests de stabilité à la température.

Les résultats obtenus démontrent que l'utilisation des copolymères selon l'invention conduisent à une compatibilité pigmentaire, ainsi qu'à une stabilité à la température systématiquement améliorées par rapport au polymère de l'art antérieur.

## Revendications

1. Utilisation de copolymères acryliques hydrosolubles dans des formulations aqueuses éventuellement pigmentées, comme épaississant permettant d'obtenir un développement et/ou un maintien de la viscosité des formulations aqueuses en présence de composés hydrosolubles ou non ou lors de l'addition des dits composés dans les dites formulations, **caractérisée en ce que** les dits copolymères sont constitués :
a) d'au moins un monomère à insaturation éthylénique et à fonction carboxylique,
b) d'au moins un monomère non ionique à insaturation éthylénique,
c) d'au moins un monomère oxyalkylé à insaturation éthylénique terminé par une chaîne ramifiée hydrophobe et non aromatique comportant de 10 à 24 atomes de carbone, et possède 2 ramifications comportant au moins 6 atomes de carbone,
d) et éventuellement d'au moins un monomère comportant au moins deux insaturations éthyléniques.

2. Utilisation selon la revendication 1 de copolymères acryliques hydrosolubles dans des formulations aqueuses éventuellement pigmentées, comme épaississant permettant d'obtenir un développement et/ou un maintien de la viscosité des formulations aqueuses en présence de composés hydrosolubles ou non ou lors de l'addition des dits composés dans les dites formulations, **caractérisée en ce que** :
a) le ou les monomères à insaturation éthylénique et à fonction carboxylique sont choisis parmi les monomères à insaturation éthylénique et à fonction monocarboxylique tels que l'acide acrylique ou méthacrylique, ou encore les hémiesters de diacides tels que les monoesters en C₁ à C₄ des acides maléique ou itaconique, ou leurs mélanges, ou choisi parmi les monomères à insaturation éthylénique et fonction dicarboxylique tels que l'acide crotonique, isocrotonique, cinnamique, itaconique, maléique, ou encore les anhydrides d'acides carboxyliques, tels que l'anhydride maléique,
b) le ou les monomères non ioniques à insaturation éthylénique son choisis parmi les esters, les amides ou les nitriles des acides acrylique et méthacrylique, tels que les acrylates ou méthacrylates de méthyle, éthyle, butyle, 2-éthyle-hexyle, ou par l'acrylonitrile, l'acétate de vinyle, le styrène, le méthylstyrène, le diisobutylène, la vinylpyrrolidone, la vinylcaprolactame,
c) le ou les monomères oxyalkylé à insaturation éthylénique et terminés par une chaîne ramifiée hydrophobe et non aromatique comportant de 10 à 24 atomes de carbone, et possède 2 ramifications comportant au moins 6 atomes de carbone, possèdent la formule suivante : 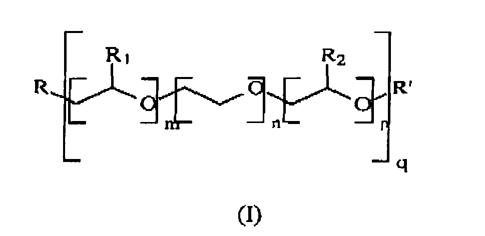 dans laquelle :
- m et p représentent un nombre de motifs d'oxyde d'alkylène allant de 0 à 150,
- n représente un nombre de motifs d'oxyde d'éthylène allant de 5 à 150,
- q représente un nombre entier au moins égal à 1 et tel que 5 ≤ (m+n+p)q ≤ 150, et préférentiellement tel que 15 ≤ (m+n+p)q ≤ 120,
- R₁ représente le radical méthyle ou éthyle,
- R₂ représente le radical méthyle ou éthyle,
- R représente un radical contenant une fonction insaturée polymérisable, appartenant au groupe des esters acrylique, méthacrylique, maléique, itaconique, ou crotonique,
- R' représente une chaîne ramifiée hydrophobe et non aromatique comportant de 10 à 24 atomes de carbone, et possède 2 ramifications comportant au moins 6 atomes de carbone.
d) le ou les monomères éventuels, appelés monomères réticulants, comportant au moins deux insaturations éthyléniques sont choisis dans le groupe constitué par le diacrylate d'éthylène glycol, le diméthacrylate d'éthylène glycol, le triméthylolpropanetriacrylate, le triméthylolpropanetriméthacrylate, l'acrylate d'allyle, le méthylène-bis-acrylamide, le méthylène-bis-méthacrylamide, le tétrallyloxyéthane, les triallylcyanurates, les éthers allyliques obtenus à partir de polyols choisis parmi le pentaérythritol, le sorbitol, ou encore le sucrose.

3. Utilisation selon l'une quelconque des revendications 1 ou 2 de copolymères acryliques hydrosolubles dans des formulations aqueuses éventuellement pigmentées, comme épaississant permettant d'obtenir un développement et/ou un maintien de la viscosité des formulations aqueuses en présence de composés hydrosolubles ou non ou lors de l'addition des dits composés dans les dites formulations, **caractérisée en ce que** les dits copolymères contiennent, exprimé en poids :
a) de 2 à 95 %, et préférentiellement de 5 à 90 % d'au moins un monomère à insaturation éthylénique et à fonction carboxylique choisis parmi les monomères à insaturation éthylénique et à fonction monocarboxylique tels que l'acide acrylique ou méthacrylique, ou encore les hémiesters de diacides tels que les monoesters en C₁ à C₄ des acides maléique ou itaconique, ou leurs mélanges, ou choisi parmi les monomères à insaturation éthylénique et fonction dicarboxylique tels que l'acide crotonique, isocrotonique, cinnamique, itaconique, maléique, ou encore les anhydrides d'acides carboxyliques, tels que l'anhydride maléique,
b) de 2 à 95 %, et préférentiellement de 5 à 90 % d'au moins un monomère non ionique à insaturation éthylénique choisis parmi les esters, les amides ou les nitriles des acides acrylique et méthacrylique, tels que les acrylates ou méthacrylates de méthyle, éthyle, butyle, 2-éthyle-hexyle, ou par l'acrylonitrile, l'acétate de vinyle, le styrène, le méthylstyrène, le diisobutylène, la vinylpyrrolidone, la vinylcaprolactame,
c) de 2 à 25 % et préférentiellement de 5 à 20 % d'au moins un monomère à insaturation éthylénique et terminé par une chaîne ramifiée hydrophobe et non aromatique comportant de 10 à 24 atomes de carbone, et possède 2 ramifications comportant au moins 6 atomes de carbone, possédant la formule suivante : 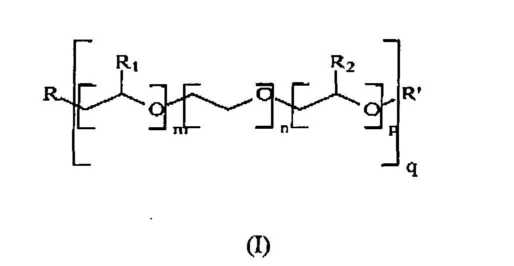 dans laquelle :
- m et p représentent un nombre de motifs d'oxyde d'alkylène allant de 0 à 150,
- n représente un nombre de motifs d'oxyde d'éthylène allant de 5 à 150,
- q représente un nombre entier au moins égal à 1 et tel que 5 ≤ (m+n+p)q ≤ 150, et préférentiellement tel que 15 ≤ (m+n+p)q ≤ 120,
- R₁ représente le radical méthyle ou éthyle,
- R₂ représente le radical méthyle ou éthyle,
- R représente un radical contenant une fonction insaturée polymérisable, appartenant au groupe des esters acrylique, méthacrylique, maléique, itaconique, ou crotonique,
- R' représente une chaîne ramifiée hydrophobe et non aromatique comportant de 10 à 24 atomes de carbone, et possède 2 ramifications comportant au moins 6 atomes de carbone.
d) de 0 % à 3 %, d'au moins un monomère comportant au moins deux insaturations éthyléniques choisis dans le groupe constitué par le diacrylate d'ethylène glycol, le diméthacrylate d'éthylène glycol, le triméthylolpropanetriacrylate, le triméthylolpropanetriméthacrylate, l'acrylate d'allyle, le méthylène-bis-acrylamide, le méthylène-bis-méthacrylamide, le tétrallyloxyéthane, les triallylcyanurates, les éthers allyliques obtenus à partir de polyols choisis parmi le pentaérythritol, le sorbitol, ou encore le sucrose,
le total en poids des monomères a), b), c) et d) étant égal à 100 %.

4. Utilisation selon la revendication 1 à 3 de copolymères acryliques hydrosolubles dans des formulations aqueuses éventuellement pigmentées, comme épaississant permettant d'obtenir un développement et/ou un maintien de la viscosité des formulations aqueuses en présence de composés hydrosolubles ou non ou lors de l'addition des dits composés dans les dites formulations, **caractérisée en ce que** la chaîne ramifiée hydrophobe et non aromatique du monomère c) comporte de 16 à 20 atomes de carbone, et possède 2 ramifications comportant au moins 6 atomes de carbone.

5. Utilisation selon la revendication 4 de copolymères acryliques hydrosolubles dans des formulations aqueuses éventuellement pigmentées, comme épaississant permettant d'obtenir un développement et/ou un maintien de la viscosité des formulations aqueuses en présence de composés hydrosolubles ou non ou lors de l'addition des dits composés dans les dites formulations, **caractérisée en ce que** la chaîne ramifiée hydrophobe et non aromatique du monomère c) est choisie parmi le 2-hexyl 1-decanyle et le 2-octyl 1-dodecanyle.

6. Utilisation selon l'une quelconque des revendications 1 à 5 de copolymères acryliques hydrosolubles dans des formulations aqueuses éventuellement pigmentées, comme épaississant permettant d'obtenir un développement et/ou un maintien de la viscosité des formulations aqueuses en présence de composés hydrosolubles ou non ou lors de l'addition des dits composés dans les dites formulations, **caractérisée en ce que** le dit copolymère ne contient pas le monomère réticulant d).

7. Utilisation selon l'une quelconque des revendications 1 à 6 de copolymères acryliques hydrosolubles dans des formulations aqueuses éventuellement pigmentées, comme épaississant permettant d'obtenir un développement et/ou un maintien de la viscosité des formulations aqueuses en présence de composés hydrosolubles ou non ou lors de l'addition des dits composés dans les dites formulations, **caractérisée en ce que** les dits copolymères sont sous leur forme acide ou partiellement ou totalement neutralisé par un ou plusieurs agents de neutralisation disposant d'une fonction neutralisante monovalente tels que ceux choisis dans le groupe constitué par les cations alcalins, en particulier le sodium, le potassium, le lithium, l'ammonium ou les amines primaires, secondaires ou tertiaires aliphatiques et/ou cycliques telles que la stéarylamine, les éthanolamines (mono-, di-, triéthanolamine), la mono et diethylamine, la cyclohexylamine, la méthylcyclohexylamine, le 2-amino 2-méthyl 1-propanol, la morpholine.

8. Utilisation selon l'une des revendications 1 à 7 de copolymères acryliques hydrosolubles dans des formulations aqueuses éventuellement pigmentées, comme épaississant permettant d'obtenir un développement et/ou un maintien de la viscosité des formulations aqueuses en présence de composés hydrosolubles ou non ou lors de l'addition des dits composés dans les dites formulations, **caractérisée en ce que** les formulations de revêtement sont choisies parmi les peintures dispersions, les vernis, les sauces de couchage papetières, la cosmétique, la détergence, les formulations textiles et les boues de forage.

9. Epaississant permettant d'obtenir un développement et/ou un maintien de la viscosité des formulations aqueuses en présence de composés hydrosolubles ou non ou lors de l'addition des dits composés dans les dites formulations, **caractérisé en ce que** le dit épaississant est un copolymère acrylique hydrosoluble constitué :
a) d'au moins un monomère à insaturation éthylénique et à fonction carboxylique,
b) d'au moins un monomère non ionique à insaturation éthylénique,
c) d'au moins un monomère oxyalkylé à insaturation éthylénique terminé par une chaîne ramifiée hydrophobe et non aromatique comportant de 10 à 24 atomes de carbone, et possède 2 ramifications comportant au moins 6 atomes de carbone,
d) et éventuellement d'au moins un monomère comportant au moins deux insaturations éthyléniques.

10. Epaississant selon la revendication 9 permettant d'obtenir un développement et/ou un maintien de la viscosité des formulations aqueuses en présence de composés hydrosolubles ou non ou lors de l'addition des dits composés dans les dites formulations, **caractérisé en ce que** :
a) le ou les monomères à insaturation éthylénique et à fonction carboxylique sont choisis parmi les monomères à insaturation éthylénique et à fonction monocarboxylique tels que l'acide acrylique ou méthacrylique, ou encore les hémiesters de diacides tels que les monoesters en C₁ à C₄ des acides maléique ou itaconique, ou leurs mélanges, ou choisi parmi les monomères à insaturation éthylénique et fonction dicarboxylique tels que l'acide crotonique, isocrotonique, cinnamique, itaconique, maléique, ou encore les anhydrides d'acides carboxyliques, tels que l'anhydride maléique,
b) le ou les monomères non ioniques à insaturation éthylénique sont choisis parmi les esters, les amides ou les nitriles des acides acrylique et méthacrylique, tels que les acrylates ou méthacrylates de méthyle, éthyle, butyle, 2-éthyle-hexyle, ou par l'acrylonitrile, l'acétate de vinyle, le styrène, le méthylstyrène, le diisobutylène, la vinylpyrrolidone, la vinylcaprolactame,
c) le ou les monomères oxyalkylé à insaturation éthylénique et terminés par une chaîne ramifiée hydrophobe et non aromatique comportant de 10 à 24 atomes de carbone, et possède 2 ramifications comportant au moins 6 atomes de carbone, possèdent la formule suivante : 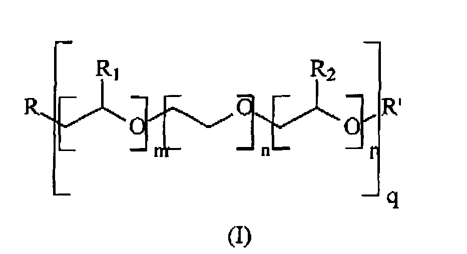 dans laquelle :
- m et p représentent un nombre de motifs d'oxyde d'alkylène allant de 0 à 150,
- n représente un nombre de motifs d'oxyde d'éthylène allant de 5 à 150,
- q représente un nombre entier au moins égal à 1 et tel que 5 ≤ (m+n+p)q ≤ 150, et préférentiellement tel que 15 ≤ (m+n+p)q ≤ 120,
- R₁ représente le radical méthyle ou éthyle,
- R₂ représente le radical méthyle ou éthyle,
- R représente un radical contenant une fonction insaturée polymérisable, appartenant au groupe des esters acrylique, méthacrylique, maléique, itaconique, ou crotonique,
- R' représente une chaîne ramifiée hydrophobe et non aromatique comportant de 10 à 24 atomes de carbone, et possède 2 ramifications comportant au moins 6 atomes de carbone,
d) le ou les monomères éventuels, appelés monomères réticulants, comportant au moins deux insaturations éthyléniques sont choisis dans le groupe constitué par le diacrylate d'éthylène glycol, le diméthacrylate d'éthylène glycol, le triméthylolpropanetriacrylate, le triméthylolpropanetriméthacrylate, l'acrylate d'allyle, le méthylène-bis-acrylamide, le méthylène-bis-méthacrylamide, le tétrallyloxyéthane, les triallylcyanurates, les éthers allyliques obtenus à partir de polyols choisis parmi le pentaérythritol, le sorbitol, ou encore le sucrose.

11. Epaississant selon la revendication 10 permettant d'obtenir un développement et/ou un maintien de la viscosité des formulations aqueuses en présence de composés hydrosolubles ou non ou lors de l'addition des dits composés dans les dites formulations, **caractérisé en ce que** le dit épaississant est un copolymère acrylique hydrosoluble constitué, exprimé en poids :
a) de 2 à 95 %, et préférentiellement de 5 à 90 % d'au moins un monomère à insaturation éthylénique et à fonction carboxylique choisis parmi les monomères à insaturation éthylénique et à fonction monocarboxylique tels que l'acide acrylique ou méthacrylique, ou encore les hémiesters de diacides tels que les monoesters en C₁ à C₄ des acides maléique ou itaconique, ou leurs mélanges, ou choisi parmi les monomères à insaturation éthylénique et fonction dicarboxylique tels que l'acide crotonique, isocrotonique, cinnamique, itaconique, maléique, ou encore les anhydrides d'acides carboxyliques, tels que l'anhydride maléique,
b) de 2 à 95 %, et préférentiellement de 5 à 90 % d'au moins un monomère non ionique à insaturation éthylénique choisis parmi les esters, les amides ou les nitriles des acides acrylique et méthacrylique, tels que les acrylates ou méthacrylates de méthyle, éthyle, butyle, 2-éthyle-hexyle, ou par l'acrylonitrile, l'acétate de vinyle, le styrène, le méthylstyrène, le diisobutylène, la vinylpyrrolidone, la vinylcaprolactame,
c) de 2 à 25 % et préférentiellement de 5 à 20 % d'au moins un monomère à insaturation éthylénique et terminé par une chaîne ramifiée hydrophobe et non aromatique comportant de 10 à 24 atomes de carbone, et possède 2 ramifications comportant au moins 6 atomes de carbone, possédant la formule suivante : 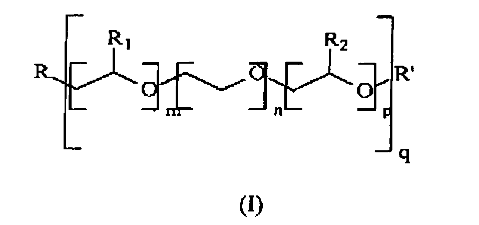 dans laquelle :
- m et p représentent un nombre de motifs d'oxyde d'alkylène allant de 0 à 150,
- n représente un nombre de motifs d'oxyde d'éthylène allant de 5 à 150,
- q représente un nombre entier au moins égal à 1 et tel que 5 ≤ (m+n+p)q ≤ 150, et préférentiellement tel que 15 ≤ (m+n+p)q ≤ 120,
- R₁ représente le radical méthyle ou éthyle,
- R₂ représente le radical méthyle ou éthyle,
- R représente un radical contenant une fonction insaturée polymérisable, appartenant au groupe des esters acrylique, méthacrylique, maléique, itaconique, ou crotonique,
- R' représente une chaîne ramifiée hydrophobe et non aromatique comportant de 10 à 24 atomes de carbone, et possède 2 ramifications comportant au moins 6 atomes de carbone.
d) de 0% à 3%, d'au moins un monomère comportant au moins deux insaturations éthyléniques choisis dans le groupe constitué par le diacrylate d'éthylène glycol, le diméthacrylate d'éthylène glycol, le triméthylolpropanetriacrylate, le triméthylolpropanetriméthacrylate, l'acrylate d'allyle, le méthylène-bis-acrylamide, le méthylène-bis-méthacrylamide, le tétrallyloxyéthane, les triallylcyanurates, les éthers allyliques obtenus à partir de polyols choisis parmi le pentaérythritol, le sorbitol, ou encore le sucrose,
le total en poids des monomères a), b), c) et d) étant égal à 100 %.

12. Epaississant selon la revendication 9 à 11 permettant d'obtenir un développement et/ou un maintien de la viscosité des formulations aqueuses en présence de composés hydrosolubles ou non ou lors de l'addition des dits composés dans les dites formulations, **caractérisé en ce que** la chaîne ramifiée hydrophobe et non aromatique du monomère c) comporte de 16 à 20 atomes de carbone, et possède 2 ramifications comportant au moins 6 atomes de carbone.

13. Epaississant selon la revendication 12 permettant d'obtenir un développement et/ou un maintien de la viscosité des formulations aqueuses en présence de composés hydrosolubles ou non ou lors de l'addition des dits composés dans les dites formulations, **caractérisé en ce que** la chaîne ramifiée hydrophobe et non aromatique du monomère c) est choisie parmi le 2-hexyl 1-decanyle et le 2-octyl 1-dodecanyle.

14. Epaississant selon l'une quelconque des revendications 9 à 13 permettant d'obtenir un développement et/ou un maintien de la viscosité des formulations aqueuses en présence de composés hydrosolubles ou non ou lors de l'addition des dits composés dans les dites formulations, **caractérisé en ce que** le dit épaississant ne contient pas le monomère réticulant d).

15. Epaississant selon l'une quelconque des revendications 9 à 14 permettant d'obtenir un développement et/ou un maintien de la viscosité des formulations aqueuses en présence de composés hydrosolubles ou non ou lors de l'addition des dits composés dans les dites formulations, **caractérisé en ce que** le dit épaississant est un copolymère acrylique hydrosoluble sous sa forme acide ou partiellement ou totalement neutralisé par un ou plusieurs agents de neutralisation disposant d'une fonction neutralisante monovalente tels que ceux choisis dans le groupe constitué par les cations alcalins, en particulier le sodium, le potassium, le lithium, l'ammonium ou les amines primaires, secondaires ou tertiaires aliphatiques et/ou cycliques telles que la stéarylamine, les
éthanolamines (mono-, di-, triéthanolamine), la mono et diéthylamine, la cyclohexylamine, la méthylcyclohexylamine, le 2-amino 2-méthyl 1-propanol, la morpholine.

16. Procédé de fabrication de formulations aqueuses éventuellement pigmentées **caractérisé en ce que** l'on ajoute aux dites formulations l'épaississant selon l'une des revendications 9 à 15 en présence de composés hydrosolubles ou non tels que des sels ou des tensio-actifs ou lors de l'addition des dits composés dans les dites formulations aqueuses.

17. Formulation aqueuse éventuellement pigmentée contenant l'épaississant selon l'une quelconque des revendications 9 à 15 .

18. Formulation aqueuse de revêtement selon la revendication 17 **caractérisée en ce qu'**elle est choisie parmi les peintures en phase aqueuse telles que les peintures dispersions, les vernis, les sauces de couchage, les formulations cosmétiques, les formulations détergentes, les formulations textiles et les boues de forage.

19. Formulation aqueuse selon la revendication 17 **caractérisée en ce qu'**elle est choisie parmi les formulations pour plaques de plâtre telles que les formulations de pâte à joint pour plaques de plâtre, les formulations pour céramique, les formulations pour cuir, les formulations de plâtre ou encore les formulations pour liants hydrauliques telles que les formulations de mortier.

## Claims

1. Use of water-soluble acrylic copolymers in aqueous formulations that may be pigmented, as a thickener for achieving an increase and/or retention of the viscosity of the aqueous formulations in the presence of compounds which are or are not water-soluble, or when adding said compounds into said formulations, **characterized in that** said copolymers are made up of:
a) at least one ethylenically-unsaturated monomer with a carboxylic function,
b) at least one non-ionic ethylenically-unsaturated monomer,
c) at least one ethylenically-unsaturated oxyalkylated monomer terminated by a branched, non-aromatic hydrophobic chain comprising 10 to 24 carbon atoms, and possessing 2 branches comprising at least 6 carbon atoms,
d) and potentially at least one monomer comprising at least two ethylenic unsaturations.

2. Use according to claim 1 of water-soluble acrylic copolymers in aqueous formulations that may be pigmented, as a thickener for achieving an increase and/or retention of the viscosity of the aqueous formulations in the presence of compounds which are or are not water-soluble, or when adding said compounds into said formulations, **characterized in that**:
a) the ethylenically-unsaturated monomers with a carboxylic function are chosen from among ethylenically-unsaturated monomers with a monocarboxylic function such as acrylic or methacrylic acid, or hemiesters of diacids such as C₁₋C₄ monoesters of maleic or itaconic acids, or mixtures thereof, or chosen from among ethylenically-unsaturated monomers with a dicarboxylic function such as crotonic, isocrotonic, cinnamic, itaconic, or maleic acids, or anhydrides of carboxylic acids, such as maleic anhydride,
b) the ethylenically-unsaturated non-ionic monomers are chosen from among esters, amides, or nitriles of acrylic and methacrylic acids, such as acrylates or methacrylates of methyl, ethyl, butyl, 2-ethyl-hexyl, or by acrylonitrile, vinyl acetate, styrene, methylstyrene, diisobutylene, vinylpyrrolidone, and vinylcaprolactame,
c) the ethylenically-unsaturated oxyalkylated monomer(s) terminated by a branched, non-aromatic hydrophobic chain comprising 10 to 24 carbon atoms, and possessing 2 branches comprising at least 6 carbon atoms, possess the following formula: 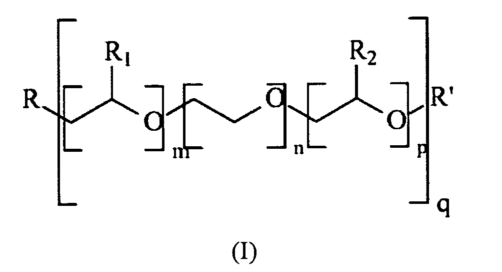 where:
- m and p represent a number of alkylene oxide units ranging from 0 to 150,
- n represents a number of ethylene oxide units ranging from 5 to 150,
- q represents an integer at least equal to 1 and such that 5 ≤ (m+n+p)q ≤ 150, and preferentially such that 15 ≤ (m+n+p)q ≤ 120,
- R₁ represents the methyl or ethyl radical,
- R₂ represents the methyl or ethyl radical,
- R represents a radical containing a polymerizable unsaturated function, belonging to the group of acrylic, methacrylic, maleic, itaconic, or crotonic esters,
- R' represents a branched, non-aromatic hydrophobic chain comprising 10 to 24 carbon atoms, and possesses 2 branches comprising at least 6 carbon atoms,
d) the monomer(s) if any, known as cross-linking monomers, comprising at least two ethylenic unsaturations are chosen from among the group constituted by ethylene glycol diacrylate, ethylene glycol dimethacrylate, ethylene glycol, trimethylolpropanetriacrylate, trimethylolpropanetrimethacrylate, allyl acrylate, methylene-bis-acrylamide, methylene-bis-methacrylamide, tetrallyloxyethane, triallylcyanurates, and allylic ethers obtained from polyols such as pentaerythritol, sorbitol, and sucrose.

3. Use according to one of the claims 1 or 2 of water-soluble acrylic copolymers in aqueous formulations that may be pigmented, as a thickener for achieving an increase and/or retention of the viscosity of the aqueous formulations in the presence of compounds which are or are not water-soluble, or when adding said compounds into said formulations, **characterized in that** said copolymers contain, expressed by weight:
a) from 2 to 95%, and preferentially from 5 to 90%, of at least one ethylenically-unsaturated monomer with a carboxylic function chosen from among ethylenically-unsaturated monomers with a monocarboxylic function such as acrylic or methacrylic acid, or hemiesters of diacids such as C₁-C₄ monoesters of maleic or itaconic acids, or mixtures thereof, or chosen from among ethylenically-unsaturated monomers with a dicarboxylic function such as crotonic, isocrotonic, cinnamic, itaconic, or maleic acids, or anhydrides of carboxylic acids, such as maleic anhydride,
b) from 2 to 95%, and preferentially from 5 to 90%, of at least one non-ionic ethylenically-unsaturated monomer chosen from among esters, amides, or nitriles of acrylic and methacrylic acids, such as acrylates or methacrylates of methyl, ethyl, butyl, 2-ethyl-hexyl, or by acrylonitrile, vinyl acetate, styrene, methylstyrene, diisobutylene, vinylpyrrolidone, and vinylcaprolactame,
c) from 2 to 25% and preferentially from 5 to 20%, of at least one ethylenically-unsaturated monomer terminated by a branched, non-aromatic hydrophobic chain comprising 10 to 24 carbon atoms, and possesses 2 branches comprising at least 6 carbon atoms, possessing the following formula: 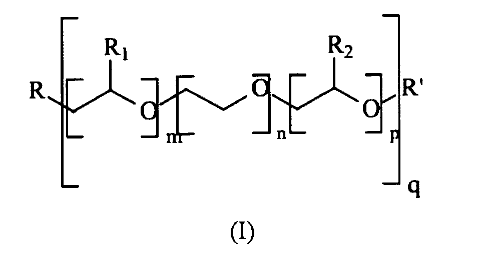 where:
- m and p represent a number of alkylene oxide units ranging from 0 to 150,
- n represents a number of ethylene oxide units ranging from 5 to 150,
- q represents an integer at least equal to 1 and such that 5 ≤ (m+n+p)q ≤ 150, and preferentially such that 15 ≤ (m+n+p)q ≤ 120,
- R₁ represents the methyl or ethyl radical,
- R₂ represents the methyl or ethyl radical,
- R represents a radical containing a polymerizable unsaturated function, belonging to the group of acrylic, methacrylic, maleic, itaconic, or crotonic esters,
- R' represents a branched, non-aromatic hydrophobic chain comprising 10 to 24 carbon atoms, and possesses 2 branches comprising at least 6 carbon atoms,
d) from 0% to 3% of at least one monomer comprising at least two ethylenic unsaturations chosen from the group constituted by ethylene glycol diacrylate, ethylene glycol dimethacrylate, ethylene glycol, trimethylolpropanetriacrylate, trimethylolpropanetrimethacrylate, allyl acrylate, methylene-bis-acrylamide, methylene-bis-methacrylamide, tetrallyloxyethane, triallylcyanurates, and allylic ethers obtained from polyols such as pentaerythritol, sorbitol, and sucrose.
the total by weight of monomers a), b), c) and d) being equal to 100%.

4. Use according to one of the claims 1 or 3 of water-soluble acrylic copolymers in aqueous formulations that may be pigmented, as a thickener for achieving an increase and/or retention of the viscosity of the aqueous formulations in the presence of compounds which are or are not water-soluble, or when adding said compounds into said formulations, **characterized in that** the branched, non-aromatic hydrophobic chain of monomer c) comprises 16 to 20 carbon atoms, and possesses 2 branches comprising at least 6 carbon atoms.

5. Use according to claim 4, of water-soluble acrylic copolymers in aqueous formulations that may be pigmented, as a thickener for achieving an increase and/or retention of the viscosity of the aqueous formulations in the presence of compounds which are or are not water-soluble, or when adding said compounds into said formulations, **characterized in that** the non-aromatic hydrophobic chain of monomer c) is chosen from among 2-hexyl 1-decanyl and 2-octyl 1-dodecanyl.

6. Use according to one of the claims 1 or 5 of water-soluble acrylic copolymers in aqueous formulations that may be pigmented, as a thickener for achieving an increase and/or retention of the viscosity of the aqueous formulations in the presence of compounds which are or are not water-soluble, or when adding said compounds into said formulations, **characterized in that** said copolymer does not contain the cross-linked monomer d).

7. Use according to one of the claims 1 or 6 of water-soluble acrylic copolymers in aqueous formulations that may be pigmented, as a thickener for achieving an increase and/or retention of the viscosity of the aqueous formulations in the presence of compounds which are or are not water-soluble, or when adding said compounds into said formulations, **characterized in that** said copolymers are in their acidic form or are partially or totally neutralized by one or more neutralization agents having a monovalent neutralizing function such as those chosen from the group constituted by alkaline cations, in particular sodium, potassium, lithium, ammonium or primary, secondary, or tertiary aliphatic and/or cyclic amines such as stearylamine, ethanolamines (mono-, di-, triethanolamine), mono- and diethylamine, cyclohexylamine, methylcyclohexylamine, 2-amino 2-methyl 1-propanol, and morpholine.

8. Use according to one of the claims 1 to 7 of water-soluble acrylic copolymers in aqueous formulations that may be pigmented, as a thickener for achieving an increase and/or retention of the viscosity of the aqueous formulations in the presence of compounds which are or are not water-soluble, or when adding said compounds into said formulations, **characterized in that** the coating formulations are chosen from among dispersion paints, varnishes, paper coating, cosmetics, detergents, textile formulations, and drilling muds.

9. A thickener for achieving an increase and/or retention of the viscosity of the aqueous formulations in the presence of compounds which are or are not water-soluble, or when adding said compounds into said formulations, **characterized in that** said thickener is a water-soluble acrylic copolymer made up of:
a) at least one ethylenically-unsaturated monomer with a carboxylic function,
b) at least one non-ionic ethylenically-unsaturated monomer,
c) at least one ethylenically-unsaturated oxyalkylated monomer terminated by a branched, non-aromatic hydrophobic chain comprising 10 to 24 carbon atoms, and possessing 2 branches comprising at least 6 carbon atoms,
d) and potentially at least one monomer comprising at least two ethylenic unsaturations.

10. A thickener according to claim 9 for achieving an increase and/or retention of the viscosity of the aqueous formulations in the presence of compounds which are or are not water-soluble, or when adding said compounds into said formulations, **characterized in that**:
a) the ethylenically-unsaturated monomers with a carboxylic function are chosen from among ethylenically-unsaturated monomers with a monocarboxylic function such as acrylic or methacrylic acid, or hemiesters of diacids such as C₁₋C₄ monoesters of maleic or itaconic acids, or mixtures thereof, or chosen from among ethylenically-unsaturated monomers with a dicarboxylic function such as crotonic, isocrotonic, cinnamic, itaconic, or maleic acids, or anhydrides of carboxylic acids, such as maleic anhydride,
b) the ethylenically-unsaturated non-ionic monomers are chosen from among esters, amides, or nitriles of acrylic and methacrylic acids, such as acrylates or methacrylates of methyl, ethyl, butyl, 2-ethyl-hexyl, or by acrylonitrile, vinyl acetate, styrene, methylstyrene, diisobutylene, vinylpyrrolidone, and vinylcaprolactame,
c) the ethylenically-unsaturated oxyalkylated monomer(s) terminated by a branched, non-aromatic hydrophobic chain comprising 10 to 24 carbon atoms, and possessing 2 branches comprising at least 6 carbon atoms, possess the following formula: 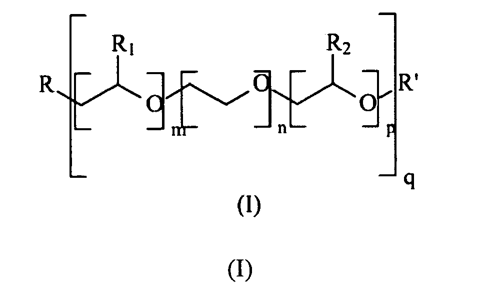 where:
- m and p represent a number of alkylene oxide units ranging from 0 to 150,
- n represents a number of ethylene oxide units ranging from 5 to 150,
- q represents an integer at least equal to 1 and such that 5 ≤ (m+n+p)q ≤ 150, and preferentially such that 15 ≤ (m+n+p)q ≤ 120,
- R₁ represents the methyl or ethyl radical,
- R₂ represents the methyl or ethyl radical,
- R represents a radical containing a polymerizable unsaturated function, belonging to the group of acrylic, methacrylic, maleic, itaconic, or crotonic esters,
- R' represents a branched, non-aromatic hydrophobic chain comprising 10 to 24 carbon atoms, and possesses 2 branches comprising at least 6 carbon atoms,
d) the monomer(s) if any, known as cross-linking monomers, comprising at least two ethylenic unsaturations are chosen from among the group constituted by ethylene glycol diacrylate, ethylene glycol dimethacrylate, ethylene glycol, trimethylolpropanetriacrylate, trimethylolpropanetrimethacrylate, allyl acrylate, methylene-bis-acrylamide, methylene-bis-methacrylamide, tetrallyloxyethane, triallylcyanurates, and allylic ethers obtained from polyols such as pentaerythritol, sorbitol, and sucrose.

11. A thickener according to claim 10 for achieving an increase and/or retention of the viscosity of the aqueous formulations in the presence of compounds which are or are not water-soluble, or when adding said compounds into said formulations, **characterized in that** said thickener is a water-soluble acrylic copolymer made up of:
a) from 2 to 95%, and preferentially from 5 to 90%, of at least one ethylenically-unsaturated monomer with a carboxylic function chosen from among ethylenically-unsaturated monomers with a monocarboxylic function such as acrylic or methacrylic acid, or hemiesters of diacids such as C₁-C₄ monoesters of maleic or itaconic acids, or mixtures thereof, or chosen from among ethylenically-unsaturated monomers with a dicarboxylic function such as crotonic, isocrotonic, cinnamic, itaconic, or maleic acids, or anhydrides of carboxylic acids, such as maleic anhydride,
b) from 2 to 95%, and preferentially from 5 to 90%, of at least one non-ionic ethylenically-unsaturated monomer chosen from among esters, amides, or nitriles of acrylic and methacrylic acids, such as acrylates or methacrylates of methyl, ethyl, butyl, 2-ethyl-hexyl, or by acrylonitrile, vinyl acetate, styrene, methylstyrene, diisobutylene, vinylpyrrolidone, and vinylcaprolactame,
c) from 2 to 25% and preferentially from 5 to 20%, of at least one ethylenically-unsaturated monomer terminated by a branched, non-aromatic hydrophobic chain comprising 10 to 24 carbon atoms, and possesses 2 branches comprising at least 6 carbon atoms, possessing the following formula: 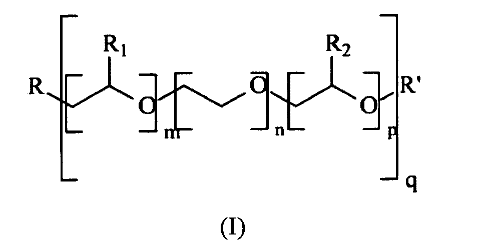 where:
- m and p represent a number of alkylene oxide units ranging from 0 to 150,
- n represents a number of ethylene oxide units ranging from 5 to 150,
- q represents an integer at least equal to 1 and such that 5 ≤ (m+n+p)q ≤ 150, and preferentially such that 15 ≤ (m+n+p)q ≤ 120,
- R₁ represents the methyl or ethyl radical,
- R₂ represents the methyl or ethyl radical,
- R represents a radical containing a polymerizable unsaturated function, belonging to the group of acrylic, methacrylic, maleic, itaconic, or crotonic esters,
- R' represents a branched, non-aromatic hydrophobic chain comprising 10 to 24 carbon atoms, and possesses 2 branches comprising at least 6 carbon atoms,
d) from 0% to 3% of at least one monomer comprising at least two ethylenic unsaturations chosen from the group constituted by ethylene glycol diacrylate, ethylene glycol dimethacrylate, ethylene glycol, trimethylolpropanetriacrylate, trimethylolpropanetrimethacrylate, allyl acrylate, methylene-bis-acrylamide, methylene-bis-methacrylamide, tetrallyloxyethane, triallylcyanurates, and allylic ethers obtained from polyols such as pentaerythritol, sorbitol, and sucrose.
the total by weight of monomers a), b), c) and d) being equal to 100%.

12. A thickener according to one of the claims 9 to 11 for achieving an increase and/or retention of the viscosity of the aqueous formulations in the presence of compounds which are or are not water-soluble, or when adding said compounds into said formulations, **characterized in that** the branched, non-aromatic hydrophobic chain of monomer c) comprises 16 to 20 carbon atoms, and possesses 2 branches comprising at least 6 carbon atoms.

13. A thickener according to claim 12 for achieving an increase and/or retention of the viscosity of the aqueous formulations in the presence of compounds which are or are not water-soluble, or when adding said compounds into said formulations, **characterized in that** the non-aromatic hydrophobic chain of monomer c) is chosen from among 2-hexyl 1-decanyl and 2-octyl 1-dodecanyl.

14. A thickener according to one of the claims 9 to 13 for achieving an increase and/or retention of the viscosity of the aqueous formulations in the presence of compounds which are or are not water-soluble, or when adding said compounds into said formulations, **characterized in that** said thickener does not contain the cross-linked monomer d).

15. A thickener according to one of the claims 9 to 14 for achieving an increase and/or retention of the viscosity of the aqueous formulations in the presence of compounds which are or are not water-soluble, or when adding said compounds into said formulations, **characterized in that** said thickener is a water-soluble acrylic copolymer in its acidic form or partially or totally neutralized by one or more neutralization agents having a monovalent neutralizing function such as those chosen from the group constituted by alkaline cations, in particular sodium, potassium, lithium, ammonium or primary, secondary, or tertiary aliphatic and/or cyclic amines such as stearylamine, ethanolamines (mono-, di-, triethanolamine), mono- and diethylamine, cyclohexylamine, methylcyclohexylamine, 2-amino 2-methyl 1-propanol, and morpholine.

16. A method for manufacturing water-soluble acrylic copolymers in aqueous formulations that may be pigmented, characteized in that thickener is added to said formulations according to one of the claims 9 to 15 in the presence of compounds which are or are not water-soluble, such as salts or surface active agents, or when adding said compounds into said aqueous formulations.

17. An aqueous formulations that may be pigmented, containing the thickener according to one of the claims 9 to 15.

18. An aqueous coating formulation according to claim 17, **characterized in that** it is chosen from among paints in an aqueous phase such as dispersion paints, varnishes, paper coating, cosmetics, detergents, textile formulations, and drilling muds.

19. An aqueous coating formulation according to claim 17, **characterized in that** it is chosen from among formulations for plasterboards such as joint compound formulations for plasterboards, formulations for ceramics, formulations for leather, plaster formulations, or formulations for hydraulic binders such as mortar formulations.

## Patentansprüche

1. Verwendung von wasserlöslichen Acryl-Copolymeren in gegebenenfalls pigmentierten wässrigen Formulierungen als Verdickungsmittel, welches eine Entwicklung und/oder ein Erhalten der Viskosität der wässrigen Formulierungen, gegebenenfalls in Gegenwart von wasserlöslichen Verbindungen oder bei Hinzufügen der besagten Verbindungen zu den besagten Formulierungen, ermöglicht, **dadurch gekennzeichnet, dass** die besagten Copolymere bestehen aus:
a) Mindestens einem ethylenisch ungesättigten Monomer mit Carbonfunktion,
b) mindestens einem nichtionischen ethylenisch ungesättigten Monomer,
c) mindestens einem oxalkylierten ethylenisch ungesättigten Monomer, terminiert durch eine verzweigte hydrophobe und nichtaromatische Kette mit 10 bis 24 Kohlenstoffatomen, wobei 2 Verzweigungen mindestens 6 Kohlenstoffatome aufweisen,
d) und gegebenenfalls mindestens einem Monomer mit mindestens zwei ethylenischen Ungesättigtheiten.

2. Verwendung, gemäß Anspruch 1, von wasserlöslichen Acryl-Copolymeren in gegebenenfalls pigmentierten wässrigen Formulierungen als Verdickungsmittel, welches eine Entwicklung und/oder ein Erhalten der Viskosität der wässrigen Formulierungen, gegebenenfalls in Gegenwart von wasserlöslichen Verbindungen oder bei Hinzufügen der besagten Verbindungen zu den besagten Formulierungen, ermöglicht, **dadurch gekennzeichnet, dass**:
a) das oder die ethylenisch ungesättigte(n) Monomer(e) mit Carbonfunktion unter den ethylenisch ungesättigten Monomeren mit einbasischer Carbonfunktion wie die Acryl- oder die Methacrylsäure oder auch den Disäure-Hemiestern wie die C₁ bis C₄-Monoester der Malein- oder Itaconsäure, oder einem Gemisch daraus, gewählt wird/werden, oder unter den ethylenisch ungesättigten Monomeren mit zweibasischer Carbonfunktion wie die Croton-, Isocroton-, Zimt-, Itacon- und Maleinsäure, oder auch den Carbonsäureanhydriden wie das Maleinanhydrid, gewählt wird/werden,
b) das oder die nicht-ionische(n) ethylenisch ungesättigte(n) Monomer(e) unter den Estern, den Amiden oder den Nitrilen der Acryl- oder Methacrylsäure wie die Methylacrylate oder -methacrylate, die Ethylacrylate oder -methacrylate, die Butylacrylate oder -methacrylate, die 2-Ethylhexylacrylate oder -methacrylate, oder unter Acrylnitril, Vinylacetat, Styrol, Methylstyrol, Diisobutylen, Vinylpyrrolidon, Vinylcaprolactam gewählt wird/werden,
c) das oder die ethylenisch ungesättigte(n) oxyalkylierte(n) Monomer(e), welche(s) durch eine verzeigte hydrophobe und nichtaromatische Kette mit 10 bis 24 Kohlenstoffatomen, wobei 2 Verzweigungen mindestens 6 Kohlenstoffatome aufweisen, terminiert ist/sind, die folgende Formel hat/haben: 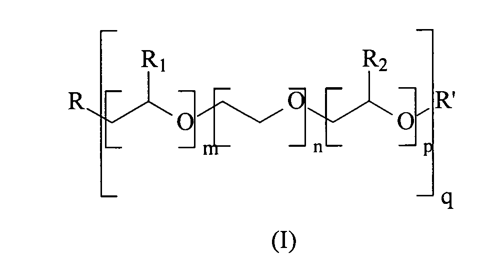 wobei:
- m und p eine Anzahl von 0 bis 150 Alkylenoxideinheiten darstellen,
- n eine Anzahl von 5 bis 150 Alkylenoxideinheiten darstellt,
- q eine ganze Zahl, welche mindestens gleich 1 wie 5 ≤ (m+n+p)q ≤ 150, und vorzugsweise wie 15 ≤ (m+n+p)q ≤ 120 ist, darstellt
- R₁ das Methyl- oder Ethylradikal darstellt,
- R₂ das Methyl- oder Ethylradikal darstellt,
- R ein Radikal darstellt, welches eine ungesättigte polymerisierbare Funktion enthält und der Gruppe der Acryl-, Methacryl-, Malein-, Itacon- oder Crotonester angehört,
- R' eine verzweigte hydrophobe und nichtaromatische Kette mit 10 bis 24 Kohlenstoffatomen, wobei 2 Verzweigungen mindestens 6 Kohlenstoffatome aufweisen, darstellt,
d) das oder die eventuelle(n) Monomer(e), vernetzende(s) Monomer(e) genannt, welche(s) mindestens zwei ethylenische Ungesättigtheiten enthält/enthalten, in der Gruppe bestehend aus Ethylenglykoldiacrylat, Ethylenglykoldimethacrylat, Trimethylolpropantriacrylat, Trimethylolpropantrimethacrylat, Allylacrylat, Methylenbisacrylamid, Methylenbismethacrylamid, Tetrallyloxyethan, Triallylcyanurat, den Allylethern, welche aus unter dem Pentaerythritol, dem Sorbitol oder auch der Sucrose gewählten Allylethern gewonnen werden, ausgewählt wird/werden.

3. Verwendung, gemäß einem beliebigen der Ansprüche 1 oder 2, von wasserlöslichen Acryl-Copolymeren in gegebenenfalls pigmentierten wässrigen Formulierungen als Verdickungsmittel, um eine Entwicklung und/oder ein Erhalten der Viskosität der wässrigen Formulierungen, gegebenenfalls in Gegenwart von wasserlöslichen Verbindungen oder bei Hinzufügen der besagten Verbindungen zu den besagten Formulierungen zu ermöglichen, **dadurch gekennzeichnet, dass** die besagten Copolymere, ausgedrückt in Gewicht, enthalten:
a) Von 2 bis 95 %, und vorzugsweise von 5 bis 90 % mindestens eines ethylenisch ungesättigten Monomers mit Carbonfunktion, gewählt unter den ethylenisch ungesättigten Monomeren mit einbasischer Carbonfunktion wie die Acryl- oder Methacrylsäure, oder auch den Disäure-Hemiestern wie die C₁ bis C₄-Monoester der Malein- oder Itaconsäure, oder Gemische daraus, oder gewählt unter den ethylenisch ungesättigten Monomeren mit zweibasischer Carbonfunktion wie Croton-, Isocroton-, Zimt-, Itacon-, Maleinsäure, oder auch den Carbonsäureanhydriden wie Maleinanhydrid,
b) von 2 bis 95 %, und vorzugsweise von 5 bis 90 % mindestens eines nichtionischen ethylenisch ungesättigten Monomers, gewählt unter den Estern, den Amiden oder den Nitrilen der Acryl- und Methacrylsäure wie die Methylacrylate oder -methacrylate, die Ethylacrylate oder -methacrylate, die Butylacrylate oder -methacrylate, die 2-Ethylhexylacrylate oder -methacrylate, oder unter Acrylnitril, Vinylacetat, Styrol, Methylstyrol, Diisobutylen, Vinylpyrrolidon, Vinylcaprolactam,
c) von 2 bis 25 % und vorzugsweise von 5 bis 20 % mindestens eines ethylenisch ungesättigten Monomers, terminiert durch eine verzweigte hydrophobe und nichtaromatische Kette mit 10 bis 24 Kohlenstoffatomen, wobei 2 Verzweigungen mindestens 6 Kohlenstoffatomen aufweisen, mit der folgenden Formel: 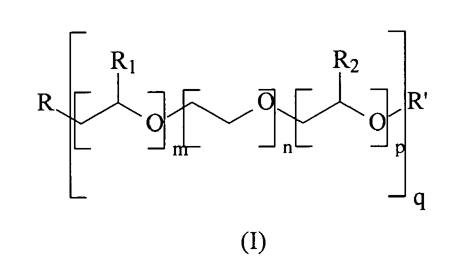 wobei:
- m und p eine Anzahl von 0 bis 150 Alkylenoxideinheiten darstellen,
- n eine Anzahl von 5 bis 150 Alkylenoxideinheiten darstellt,
- q eine ganze Zahl, welche mindestens gleich 1 wie 5 ≤ (m+n+p)q ≤ 150, und vorzugsweise wie 15 ≤ (m+n+p)q ≤ 120 ist, darstellt
- R₁ das Methyl- oder Ethylradikal darstellt,
- R₂ das Methyl- oder Ethylradikal darstellt,
- R ein Radikal darstellt, welches eine ungesättigte polymerisierbare Funktion enthält und der Gruppe der Acryl-, Methacryl-, Malein-, Itacon- oder Crotonester angehört,
- R' eine verzweigte hydrophobe und nichtaromatische Kette mit 10 bis 24 Kohlenstoffatomen, wobei 2 Verzweigungen mindestens 6 Kohlenstoffatome aufweisen, darstellt,
d) von 0 % bis 3 % mindestens eines Monomers mit mindestens zwei ethylenischen Ungesättigtheiten, gewählt in der Gruppe bestehend aus Ethylenglykoldiacrylat, Ethylenglykoldimethacrylat, Trimethylolpropantriacrylat, Trimethylolpropantrimethacrylat, Allylacrylat, Methylenbisacrylamid, Methylenbismethacrylamid, Tetrallyloxyethan, Triallylcyanurat, den Allylethern, welche aus unter dem Pentaerythritol, dem Sorbitol oder auch der Sucrose gewählten Allylethern gewonnen werden,
wobei das Gesamtgewicht der Monomere a), b), c) und d) gleich 100 % ist.

4. Verwendung, gemäß Anspruch 1 bis 3, von wasserlöslichen Acryl-Copolymeren in gegebenenfalls pigmentierten wässrigen Formulierungen als Verdickungsmittel, um eine Entwicklung und/oder ein Erhalten der Viskosität der wässrigen Formulierungen, gegebenenfalls in Gegenwart von wasserlöslichen Verbindungen oder bei Hinzufügen der besagten Verbindungen zu den besagten Formulierungen, zu ermöglichen, **dadurch gekennzeichnet, dass** die verzweigte hydrophobe und nichtaromatische Kette des Monomers c) 16 bis 20 Kohlenstoffatome enthält, wobei 2 Verzweigungen mindestens 6 Kohlenstoffatome aufweisen.

5. Verwendung, gemäß Anspruch 4, von wasserlöslichen Acryl-Copolymeren in gegebenenfalls pigmentierten wässrigen Formulierungen als Verdickungsmittel, um eine Entwicklung und/oder ein Erhalten der Viskosität der wässrigen Formulierungen, gegebenenfalls in Gegenwart von wasserlöslichen Verbindungen oder bei Hinzufügen der besagten Verbindungen zu den besagten Formulierungen, zu ermöglichen, **dadurch gekennzeichnet, dass** die verzweigte hydrophobe und nichtaromatische Kette des Monomers c) unter dem 2-Hexyl 1-Decanol und dem 2-Octyl 1-Dodecanol gewählt wird.

6. Verwendung, gemäß einem beliebigen der Ansprüche 1 bis 5, von wasserlöslichen Acryl-Copolymeren in gegebenenfalls pigmentierten wässrigen Formulierungen als Verdickungsmittel, um eine Entwicklung und/oder ein Erhalten der Viskosität der wässrigen Formulierungen, gegebenenfalls in Gegenwart von wasserlöslichen Verbindungen oder bei Hinzufügen der besagten Verbindungen zu den besagten Formulierungen, zu ermöglichen, **dadurch gekennzeichnet, dass** das besagte Copolymer nicht das vernetzende Monomer d) enthält.

7. Verwendung, gemäß einem beliebigen der Ansprüche 1 bis 6, von wasserlöslichen Acryl-Copolymeren in gegebenenfalls pigmentierten wässrigen Formulierungen als Verdickungsmittel, um eine Entwicklung und/oder ein Erhalten der Viskosität der wässrigen Formulierungen, gegebenenfalls in Gegenwart von wasserlöslichen Verbindungen oder bei Hinzufügen der besagten Verbindungen zu den besagten Formulierungen, zu ermöglichen, **dadurch gekennzeichnet, dass** die besagten Copolymere in ihrer Säureform oder teilweise oder vollständig neutralisiert durch ein oder mehrere Neutralisationsmittel mit einer einwertigen Neutralisierungsfunktion, wie solche gewählt aus der Gruppe bestehend aus den Alkalikationen, insbesondere das Natrium, das Kalium, das Lithium, das Ammonium oder die primären, sekundären oder tertiären aliphatischen und/oder cyclischen Amine wie das Stearylamin, die Ethanolamine (Mono-, Di-, Triethanolamine), das Mono- und das Diethylamin, das Cyclohexylamin, das Methylcyclohexylamin, das 2-Amino 2-Methyl 1-Propanol, das Morpholin sind.

8. Verwendung nach einem der Ansprüche 1 bis 7, von wasserlöslichen Acryl-Copolymeren in gegebenenfalls pigmentierten wässrigen Formulierungen als Verdickungsmittel, um eine Entwicklung und/oder ein Erhalten der Viskosität der wässrigen Formulierungen, gegebenenfalls in Gegenwart von wasserlöslichen Verbindungen oder bei Hinzufügen der besagten Verbindungen zu den besagten Formulierungen, zu ermöglichen, **dadurch gekennzeichnet, dass** die Beschichtungsformulierungen unter den Dispersionsfarben, den Lacken, den Papierstreichmassen, der Kosmetik, den Wasch- und Reinigungsmitteln, den Textilformulierungen und den Bohrschlämme gewählt werden.

9. Verdickungsmittel, welches eine Entwicklung und/oder ein Erhalten der Viskosität der wässrigen Formulierungen, gegebenenfalls in Gegenwart von wasserlöslichen Verbindungen oder bei Hinzufügen der besagten Verbindungen zu den besagten Formulierungen, ermöglicht, **dadurch gekennzeichnet, dass** das besagte Verdickungsmittel ein wasserlösliches Acrylpolymer ist, bestehend aus:
a) Mindestens einem ethylenisch ungesättigten Monomer mit Carbonfunktion,
b) mindestens einem nichtionischen ethylenisch ungesättigten Monomer,
c) mindestens einem ethylenisch ungesättigten oxyalkylierten Monomer, terminiert durch eine verzweigte hydrophobe und nichtaromatische Kette mit 10 bis 24 Kohlenstoffatomen, wobei 2 Verzweigungen mindestens 6 Kohlenstoffatome aufweisen,
d) und gegebenenfalls mindestens einem Monomer mit mindestens zwei ethylenischen Ungesättigtheiten.

10. Verdickungsmittel nach Anspruch 9, welches eine Entwicklung und/oder ein Erhalten der Viskosität der wässrigen Formulierungen, gegebenenfalls in Gegenwart von wasserlöslichen Verbindungen oder bei Hinzufügen der besagten Verbindungen zu den besagten Formulierungen, ermöglicht, **dadurch gekennzeichnet, dass**:
a) das oder die ethylenisch ungesättigte(n) Monomer(e) mit Carbonfunktion gewählt werden unter den ethylenisch ungesättigten Monomeren mit einbasischer Carbonfunktion wie die Acryl- oder Methacrylsäure, oder auch unter den Disäure-Hemiestern wie die C₁ bis C₄-Monoester der Malein- oder Itaconsäure, oder Gemische daraus, oder gewählt unter den ethylenisch ungesättigten Monomeren mit zweibasischer Carbonfunktion wie Croton-, Isocroton-, Zimt-, Itacon-, Maleinsäure, oder auch den Carbonsäureanhydriden wie Maleinanhydrid,
b) das oder die nichtionische(n) ethylenisch ungesättigte(n) Monomer(e) unter den Estern, den Amiden oder den Nitrilen der Acryl- und Methacrylsäure wie die Methylacrylate oder -methacrylate, die Ethylacrylate oder -methacrylate, die Butylacrylate oder -methacrylate, die 2-Ethylhexylacrylate oder -methacrylate, oder unter Acrylnitril, Vinylacetat, Styrol, Methylstyrol, Diisobutylen, Vinylpyrrolidon, Vinylcaprolactam gewählt wird/werden,
c) das oder die ethylenisch ungesättigte(n) oxyalkylierte(n) Monomer(e), terminiert durch eine verzweigte hydrophobe und nichtaromatische Kette mit 10 bis 24 Kohlenstoffatomen, wobei 2 Verzweigungen mindestens 6 Kohlenstoffatome aufweisen, die folgende Formel hat/haben: 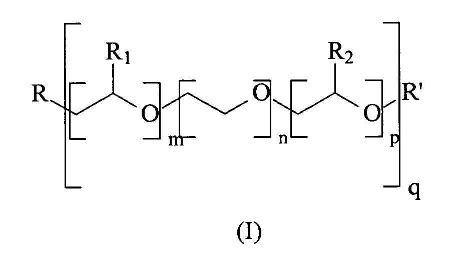 wobei:
- m und p eine Anzahl von 0 bis 150 Alkylenoxideinheiten darstellen,
- n eine Anzahl von 5 bis 150 Alkylenoxideinheiten darstellt,
- q eine ganze Zahl, welche mindestens gleich 1 wie 5 ≤ (m+n+p)q ≤ 150, und vorzugsweise wie 15 ≤ (m+n+p)q ≤ 120 ist, darstellt
- R₁ das Methyl- oder Ethylradikal darstellt,
- R₂ das Methyl- oder Ethylradikal darstellt,
- R ein Radikal darstellt, welches eine ungesättigte polymerisierbare Funktion enthält und der Gruppe der Acryl-, Methacryl-, Malein-, Itacon- oder Crotonester angehört,
- R' eine verzweigte hydrophobe und nichtaromatische Kette mit 10 bis 24 Kohlenstoffatomen, wobei 2 Verzweigungen mindestens 6 Kohlenstoffatome aufweisen, darstellt,
d) das oder die eventuelle(n) Monomer(e), vemetzende(s) Monomer(e) genannt, welche(s) mindestens zwei ethylenische Ungesättigtheiten enthält/enthalten, in der Gruppe bestehend aus Ethylenglykoldiacrylat, Ethylenglykoldimethacrylat, Trimethylolpropantriacrylat, Trimethylolpropantrimethacrylat, Allylacrylat, Methylenbisacrylamid, Methylenbismethacrylamid, Tetrallyloxyethan, Triallylcyanurat, den Allylethern, welche aus unter dem Pentaerythritol, dem Sorbitol oder auch der Sucrose gewählten Allylethern gewonnen werden, ausgewählt wird/werden.

11. Verdickungsmittel nach Anspruch 10, welches eine Entwicklung und/oder ein Erhalten der Viskosität der wässrigen Formulierungen, gegebenenfalls in Gegenwart von wasserlöslichen Verbindungen oder bei Hinzufügen der besagten Verbindungen zu den besagten Formulierungen, ermöglicht, **dadurch gekennzeichnet, dass** das Verdickungsmittel ein wasserlösliches Acrylpolymer ist, welches, ausgedrückt in Gewicht, besteht aus:
a) Von 2 bis 95 %, und vorzugsweise von 5 bis 90 % mindestens eines ethylenisch ungesättigten Monomers mit Carbonfunktion, gewählt unter den ethylenisch ungesättigten Monomeren mit einbasischer Carbonfunktion wie die Acryl- oder Methacrylsäure, oder auch den Disäure-Hemiestern wie die C₁ bis C₄-Monoester der Malein- oder Itaconsäure, oder Gemische daraus, oder gewählt unter den ethylenisch ungesättigten Monomeren mit zweibasischer Carbonfunktion wie Croton-, Isocroton-, Zimt-, Itacon-, Maleinsäure, oder auch den Carbonsäureanhydriden wie Maleinanhydrid,
b) von 2 bis 95 %, und vorzugsweise von 5 bis 90 % mindestens eines nichtionischen ethylenisch ungesättigten Monomers, gewählt unter den Estern, den Amiden oder den Nitrilen der Acryl- und Methacrylsäure wie die Methylacrylate oder -methacrylate, die Ethylacrylate oder -methacrylate, die Butylacrylate oder -methacrylate, die 2-Ethylhexylacrylate oder -methacrylate, oder unter Acrylnitril, Vinylacetat, Styrol, Methylstyrol, Diisobutylen, Vinylpyrrolidon, Vinylcaprolactam,
c) von 2 bis 25 % und vorzugsweise von 5 bis 20 % mindestens eines ethylenisch ungesättigten Monomers, terminiert durch eine verzweigte hydrophobe und nichtaromatische Kette mit 10 bis 24 Kohlenstoffatomen, wobei 2 Verzweigungen mindestens 6 Kohlenstoffatomen aufweisen, mit der folgenden Formel: 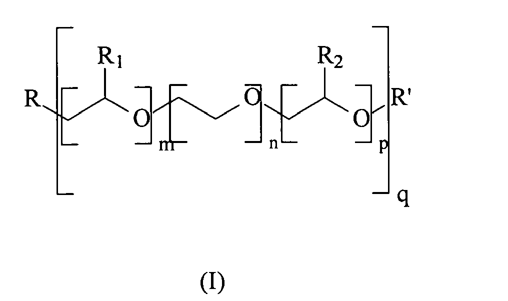 wobei:
- m und p eine Anzahl von 0 bis 150 Alkylenoxideinheiten darstellen,
- n eine Anzahl von 5 bis 150 Alkylenoxideinheiten darstellt,
- q eine ganze Zahl, welche mindestens gleich 1 wie 5 ≤ (m+n+p)q ≤ 150, und vorzugsweise wie 15 ≤ (m+n+p)q ≤ 120 ist, darstellt
- R₁ das Methyl- oder Ethylradikal darstellt,
- R₂ das Methyl- oder Ethylradikal darstellt,
- R ein Radikal darstellt, welches eine ungesättigte polymerisierbare Funktion enthält und der Gruppe der Acryl-, Methacryl-, Malein-, Itacon- oder Crotonester angehört,
- R' eine verzweigte hydrophobe und nichtaromatische Kette mit 10 bis 24 Kohlenstoffatomen, wobei 2 Verzweigungen mindestens 6 Kohlenstoffatome aufweisen, darstellt,
d) von 0 % bis 3 % mindestens eines Monomers mit mindestens zwei ethylenischen Ungesättigtheiten, gewählt in der Gruppe bestehend aus Ethylenglykoldiacrylat, Ethylenglykoldimethacrylat, Trimethylolpropantriacrylat, Trimethylolpropantrimethacrylat, Allylacrylat, Methylenbisacrylamid, Methylenbismethacrylamid, Tetrallyloxyethan, Triallylcyanurat, den Allylethern, welche aus unter dem Pentaerythritol, dem Sorbitol oder auch der Sucrose gewählten Allylethern gewonnen werden,
wobei das Gesamtgewicht der Monomere a), b), c) und d) gleich 100 % ist.

12. Verdickungsmittel nach den Ansprüchen 9 bis 11, welches eine Entwicklung und/oder ein Erhalten der Viskosität der wässrigen Formulierungen, gegebenenfalls in Gegenwart von wasserlöslichen Verbindungen oder bei Hinzufügen der besagten Verbindungen zu den besagten Formulierungen, ermöglicht, **dadurch gekennzeichnet, dass** die verzweigte hydrophobe und nichtaromatische Kette des Monomers c) 16 bis 20 Kohlenstoffatome enthält, wobei 2 Verzweigungen mindestens 6 Kohlenstoffatome aufweisen.

13. Verdickungsmittel nach Anspruch 12, welches eine Entwicklung und/oder ein Erhalten der Viskosität der wässrigen Formulierungen, gegebenenfalls in Gegenwart von wasserlöslichen Verbindungen oder bei Hinzufügen der besagten Verbindungen zu den besagten Formulierungen, ermöglicht, **dadurch gekennzeichnet, dass** die verzweigte hydrophobe und nichtaromatische Kette des Monomers c) unter dem 2-Hexyl 1-Decanol und dem 2-Octyl 1-Dodecanol gewählt wird.

14. Verdickungsmittel nach einem beliebigen der Ansprüche 9 bis 13, welches eine Entwicklung und/oder ein Erhalten der Viskosität der wässrigen Formulierungen, gegebenenfalls in Gegenwart von wasserlöslichen Verbindungen oder bei Hinzufügen der besagten Verbindungen zu den besagten Formulierungen, ermöglicht, **dadurch gekennzeichnet, dass** das besagte Verdickungsmittel nicht das vernetzende Monomer d) enthält.

15. Verdickungsmittel nach einem beliebigen der Ansprüche 9 bis 14, welches eine Entwicklung und/oder ein Erhalten der Viskosität der wässrigen Formulierungen, gegebenenfalls in Gegenwart von wasserlöslichen Verbindungen oder bei Hinzufügen der besagten Verbindungen zu den besagten Formulierung, ermöglicht, **dadurch gekennzeichnet, dass** das besagte Verdickungsmittel ein wasserlösliches AcrylCopolymer in seiner Säureform oder teilweise oder vollständig neutralisiert durch ein oder mehrere Neutralisationsmittel mit einer einwertigen Neutralisierungsfunktion, wie solche gewählt aus der Gruppe bestehend aus den Alkalikationen, insbesondere das Natrium, das Kalium, das Lithium, das Ammonium oder die primären, sekundären oder tertiären aliphatischen und/oder cyclischen Amine wie das Stearylamin, die Ethanolamine (Mono-, Di-, Triethanolamine), das Mono- und das Diethylamin, das Cyclohexylamin, das Methylcyclohexylamin, das 2-Amino 2-Methyl 1-Propanol, das Morpholin ist.

16. Verfahren zur Herstellung von gegebenenfalls pigmentierten wässrigen Formulierungen, **dadurch gekennzeichnet, dass** man den besagten Formulierungen das Verdickungsmittel gemäß einem der Ansprüche 9 bis 15 zusetzt, gegebenenfalls in Gegenwart von wasserlöslichen Verbindungen wie Salze oder Tenside oder bei Hinzufügen der besagten Verbindungen zu den besagten wässrigen Formulierungen.

17. Wässrige Formulierung, gegebenenfalls pigmentiert, welche das Verdickungsmittel gemäß einem beliebigen der Ansprüche 9 bis 15 enthält.

18. Wässrige Beschichtungsformulierung nach Anspruch 17, **dadurch gekennzeichnet, dass** sie unter den wässrigen Farben wie die Dispersionsfarben, die Lacke, die Streichmassen, die kosmetischen Formulierungen, die Wasch- und Reinigungsmittelformulierungen, die Textilformulierungen und die Bohrschlämme gewählt wird.

19. Wässrige Formulierung nach Anspruch 17, **dadurch gekennzeichnet, dass** sie unter den Formulierungen für Gipsplatten wie die Dichtungskittformulierungen für Gipsplatten, die Formulierungen für Keramik, die Formulierungen für Leder, die Gipsformulierungen oder auch unter den Formulierungen für hydraulische Bindemittel wie die Mörtelformulierungen gewählt wird.
